# EUROPEAN PATENT APPLICATION

(11) **EP 4 582 449 A1**
(43) Date of publication of application: **09.07.2025**
(21) Application number: 23859271.1
(22) Date of filing: 25.08.2023
(51) Int. Cl.: C07K 16/28, C07K 19/00, C12N 15/13, C12N 15/62, C12N 5/10, A61K 39/395, A61P 35/00, A61K 39/00

(54) **RECOMBINANT HUMANIZED MONOCLONAL ANTIBODY TARGETING HUMAN GPRC5D AND APPLICATION THEREOF**

(30) Priority: 30.08.2022 CN 202211058201
(71) Applicant: Suzhou Dima Biotechnology Co., Ltd, Suzhou, Jiangsu 215163 (CN)
(72) Inventor: MA, Donghui, Suzhou, Jiangsu 215163 (CN); ZENG, Kaikai, Suzhou, Jiangsu 215163 (CN); HE, Antao, Suzhou, Jiangsu 215163 (CN); HONG, Shujuan, Suzhou, Jiangsu 215163 (CN); MEI, Fen, Suzhou, Jiangsu 215163 (CN)
(74) Representative: dompatent von Kreisler Selting Werner - Partnerschaft von Patent- und Rechtsanwälten mbB
(86) International application number: PCT/CN2023/115089
(87) International publication number: WO 2024/046239

(57) **Abstract**

The present invention relates to a specific recombinant humanized monoclonal antibody targeting human GPRC5D and an application thereof, can be used in ELISA and flow detection methods, and can be used for diagnosis and treatment targeting GPRC5D protein. The monoclonal antibody comprises a heavy chain variable region and a light chain variable region, and the amino acid sequence and nucleotide sequence of the monoclonal antibody can be used alone or in double-stranded combination. In addition, the sequences of the specific recombinant humanized monoclonal antibody targeting human GPRC5D can be constructed into biological materials or preparations such as monoclonal antibodies, antibody conjugates, bispecific antibodies, CAR-immune cells, etc., and can be used for the diagnosis and treatment of GPRC5D-related diseases.

## Description

### Technical Field

The present invention belongs to the field of biotechnology, specifically involving a recombinant humanized monoclonal antibody targeting human GPRC5D and application thereof.

### Background

GPRC5D is an orphan receptor belonging to the G protein-coupled receptor (GPCR) family, specifically subtype D of the GPRC5 family. It is a seven-transmembrane protein. An orphan receptor refers to a receptor that is structurally similar to other known receptors but whose endogenous ligand has not yet been identified. GPRC5D is highly expressed on the surface of primary multiple myeloma (MM) cells, while its expression in normal tissues is limited to the hair follicle region. Studies have shown that 65% of multiple myeloma patients exhibit more than 50% expression of GPRC5D, making it a potential target for MM therapy. Due to the high expression of GPRC5D on the surface of plasma cells and multiple myeloma cells, and the absence of expression in other normal tissue cells and hematopoietic stem cells, GPRC5D serves as an ideal target for the diagnosis and treatment of multiple myeloma (MM).

### Summary of the Invention

The objective of the present invention is to provide a specific recombinant humanized monoclonal antibody targeting human GPRC5D. The recombinant humanized monoclonal antibody targeting human GPRC5D provided by the invention has high affinity and good specificity, which can fill the gap in the market for monoclonal antibodies targeting human GPRC5D protein in diagnostic and therapeutic applications. In addition, the GPRC5D antibody sequence can be constructed into a Chimeric Antigen Receptor T-cell (CAR-T) immunotherapy, a bispecific antibody, or an antibody-drug conjugate, which can be used for the treatment of cancer and other tumor diseases in subsequent applications.

In the first aspect of the present invention, an antibody or an antigen-binding fragment thereof is provided, which comprises a heavy chain variable region (VH) comprising the following CDRs:
VH-CDR1 as shown in SEQ ID NO: 44, 78, 1, 33, 67, 22, or 87,
VH-CDR2 as shown in SEQ ID NO: 45, 59, 79, 12, 34, 68, 2, 23, 54, or 88, and
VH-CDR3 as shown in SEQ ID NO: 46, 60, 13, 35, 69, 3, 24, 55, or 89;
wherein the antibody or its antigen-binding fragment specifically binds to GPRC5D, preferably human GPRC5D.

In another preferred embodiment, the heavy chain variable region of the antibody or its antigen-binding fragment comprises the following three CDRs:
VH-CDR1 as shown in SEQ ID NO: 44,
VH-CDR2 as shown in SEQ ID NO: 45, and
VH-CDR3 as shown in SEQ ID NO: 46.

In another preferred embodiment, the heavy chain variable region of the antibody or its antigen-binding fragment comprises the following three CDRs:
VH-CDR1 as shown in SEQ ID NO: 44,
VH-CDR2 as shown in SEQ ID NO: 59, and
VH-CDR3 as shown in SEQ ID NO: 60.

In another preferred embodiment, the heavy chain variable region of the antibody or its antigen-binding fragment comprises the following three CDRs:
VH-CDR1 as shown in SEQ ID NO: 78,
VH-CDR2 as shown in SEQ ID NO: 79, and
VH-CDR3 as shown in SEQ ID NO: 60.

In another preferred embodiment, the heavy chain variable region of the antibody or its antigen-binding fragment comprises the following three CDRs:
VH-CDR1 as shown in SEQ ID NO: 1,
VH-CDR2 as shown in SEQ ID NO: 12, and
VH-CDR3 as shown in SEQ ID NO: 13.

In another preferred embodiment, the antibody or its antigen-binding fragment further includes a light chain variable region (VL), wherein the light chain variable region comprises the following CDRs:
VL-CDR1 as shown in SEQ ID NO: 47, 61, 80, 14, 36, 70, 4, 25, 47, or 90,
VL-CDR2 as shown in SEQ ID NO: 37, 15, 71, 5, or 26, and
VL-CDR3 as shown in SEQ ID NO: 48, 81, 16, 38, 72, 6, 27, 48, or 91.

In another preferred embodiment, the light chain variable region of the antibody or its antigen-binding fragment comprises the following three CDRs:
VL-CDR1 as shown in SEQ ID NO: 47,
VL-CDR2 as shown in SEQ ID NO: 37, and
VL-CDR3 as shown in SEQ ID NO: 48.

In another preferred embodiment, the light chain variable region of the antibody or its antigen-binding fragment comprises the following three CDRs:
VL-CDR1 as shown in SEQ ID NO: 61,
VL-CDR2 as shown in SEQ ID NO: 37, and
VL-CDR3 as shown in SEQ ID NO: 48.

In another preferred embodiment, the light chain variable region of the antibody or its antigen-binding fragment comprises the following three CDRs:
VL-CDR1 as shown in SEQ ID NO: 80,
VL-CDR2 as shown in SEQ ID NO: 37, and
VL-CDR3 as shown in SEQ ID NO: 81.

In another preferred embodiment, the light chain variable region of the antibody or its antigen-binding fragment comprises the following three CDRs:
VL-CDR1 as shown in SEQ ID NO: 14,
VL-CDR2 as shown in SEQ ID NO: 15, and
VL-CDR3 as shown in SEQ ID NO: 16.

In another preferred embodiment, the antibody or its antigen-binding fragment comprises a heavy chain variable region CDR and a light chain variable region CDR selected from the following group:

| No. | VH-CDR1 Sequence ID | VH-CDR2 Sequence ID | VH-CDR3 Sequence ID | VL-CDR1 Sequence ID | VL-CDR2 Sequence ID | VL-CDR3 Sequence ID |
|---|---|---|---|---|---|---|
| G05 | 44 | 45 | 46 | 47 | 37 | 48 |
| G07 | 44 | 59 | 60 | 61 | 37 | 48 |
| G09 | 78 | 79 | 60 | 80 | 37 | 81 |
| G02 | 1 | 12 | 13 | 14 | 15 | 16 |
| G04 | 33 | 34 | 35 | 36 | 37 | 38 |
| G08 | 67 | 68 | 69 | 70 | 71 | 72 |
| G01 | 1 | 2 | 3 | 4 | 5 | 6 |
| G03 | 22 | 23 | 24 | 25 | 26 | 27 |
| G06 | 44 | 54 | 55 | 47 | 37 | 48 |
| G10 | 87 | 88 | 89 | 90 | 26 | 91. |

In another preferred embodiment, the antibody or its antigen-binding fragment comprises a heavy chain variable region with an amino acid sequence that has at least 80%, 85%, 90%, 91%, 92%, 93%, 94%, 95%, 96%, 97%, 98%, or 99% identity to SEQ ID NO: 49, 62, 82, 17, 39, 73, 7, 28, 56, or 92.

In another preferred embodiment, the antibody or its antigen-binding fragment comprises a light chain variable region with an amino acid sequence that has at least 80%, 85%, 90%, 91%, 92%, 93%, 94%, 95%, 96%, 97%, 98%, or 99% identity to SEQ ID NO: 51, 64, 84, 19, 41, 75, 9, 30, or 94.

In another preferred embodiment, the antibody or its antigen-binding fragment comprises a heavy chain variable region and a light chain variable region selected from the following group:

| No. | VH Sequence ID | VL Sequence ID |
|---|---|---|
| G05 | 49 | 51 |
| G07 | 62 | 64 |
| G09 | 82 | 84 |
| G02 | 17 | 19 |
| G04 | 39 | 41 |
| G08 | 73 | 75 |
| G01 | 7 | 9 |
| G03 | 28 | 30 |
| G06 | 56 | 51 |
| G10 | 92 | 94. |

In another preferred embodiment, the antibody or its antigen-binding fragment comprises a heavy chain variable region with an amino acid sequence as shown in SEQ ID NO: 49, and a light chain variable region with an amino acid sequence as shown in SEQ ID NO: 51.

In another preferred embodiment, the antibody or its antigen-binding fragment comprises a heavy chain variable region with an amino acid sequence as shown in SEQ ID NO: 62, and a light chain variable region with an amino acid sequence as shown in SEQ ID NO: 64.

In another preferred embodiment, the antibody or its antigen-binding fragment comprises a heavy chain variable region with an amino acid sequence as shown in SEQ ID NO: 82, and a light chain variable region with an amino acid sequence as shown in SEQ ID NO: 84.

In another preferred embodiment, the antibody or its antigen-binding fragment comprises a heavy chain variable region with an amino acid sequence as shown in SEQ ID NO: 17, and a light chain variable region with an amino acid sequence as shown in SEQ ID NO: 19.

In another preferred embodiment, the antibody includes a single-chain antibody (scFv), a dual-chain antibody, a monoclonal antibody, a chimeric antibody (such as a human-mouse chimeric antibody), an animal-derived antibody, a fully human antibody, or a humanized antibody.

In another preferred embodiment, the antibody is a humanized antibody.

In another preferred embodiment, the antibody is a monoclonal antibody.

In another preferred embodiment, the constant region of the antibody's light chain is a κ chain, and the constant region of the heavy chain is of the IgG type.

In another preferred embodiment, the antibody is a bispecific antibody or a multispecific antibody.

In the second aspect of the present invention, a recombinant protein is provided, which comprises:(i) the antibody or its antigen-binding fragment of the first aspect of the present invention; and
(ii) an optional tag sequence to assist with expression and/or purification.

In another preferred embodiment, the tag sequence is selected from the following group: 6×His tag, GGGS sequence, FLAG tag.

In the third aspect of the present invention, a polynucleotide is provided, which encodes a polypeptide selected from the following group:
(1) the antibody or its antigen-binding fragment of the first aspect of the present invention; or
(2) the recombinant protein as described in the second aspect of the present invention.

In the fourth aspect of the present invention, a vector is provided, which contains the polynucleotide of the third aspect of the present invention.

In another preferred embodiment, the vector includes: bacterial plasmids, phages, yeast plasmids, plant cell viruses, mammalian cell viruses such as adenovirus, adeno-associated virus, retroviruses, lentiviral vectors, or other vectors.

In the fifth aspect of the present invention, a genetically engineered host cell is provided, which contains the vector of the fourth aspect of the present invention, or has integrated the polynucleotide of the third aspect of the present invention into its genome.

In the sixth aspect of the present invention, an immunoconjugate is provided, which comprises:
(a) the antibody or its antigen-binding fragment of the first aspect of the present invention, or the recombinant protein of the second aspect of the present invention; and
(b) a coupled moiety selected from the group consisting of: detectable markers, drugs, toxins, cytokines, radioactive isotopes, and enzymes.

In another preferred embodiment, the coupled moiety is selected from: fluorescent or luminescent markers, radioactive markers, MRI (magnetic resonance imaging) or CT (computed tomography) contrast agents, or enzymes, radioactive isotopes, biotoxins, cytokines (such as IL-2), antibodies, antibody Fc fragments, antibody scFv fragments, gold nanoparticles/nanorods, viral particles, liposomes, magnetic nanoparticles, prodrug-activating enzymes (e.g., DT-Diaphorase (DTD) or biphenylhydrolase-like protein (BPHL)), chemotherapeutic agents (e.g., cisplatin), or any form of nanoparticles, etc.

In the seventh aspect of the present invention, a chimeric antigen receptor (CAR) is provided, wherein the antigen-binding domain of the chimeric antigen receptor comprises the antibody or its antigen-binding fragment of the first aspect of the present invention.

In the eighth aspect of the present invention, an immune cell is provided, which expresses or has the antibody or its antigen-binding fragment of the first aspect of the present invention exposed on its cell membrane.

In another preferred embodiment, the immune cell includes T cells, NK cells, or macrophages.

In another preferred embodiment, the immune cell is a CAR-T cell.

In the ninth aspect of the present invention, a pharmaceutical composition is provided, which comprises:
(z1) an active ingredient, selected from the following group: the antibody or its antigen-binding fragment of the first aspect of the present invention, the recombinant protein of the second aspect of the present invention, the immunoconjugate of the sixth aspect of the present invention, or the immune cells of the eighth aspect of the present invention, or a combination thereof; and
(z2) a pharmaceutically acceptable carrier.

In another preferred embodiment, the pharmaceutical composition is a liquid formulation.

In another preferred embodiment, the pharmaceutical composition is an injectable preparation.

In another preferred embodiment, the pharmaceutical composition is used to prepare a drug for treating diseases associated with high expression of GPRC5D.

In another preferred embodiment, the disease associated with high expression of GPRC5D is cancer, such as multiple myeloma.

In the tenth aspect of the present invention, the use of an active ingredient is provided, wherein the active ingredient is selected from the following group: the antibody or its antigen-binding fragment of the first aspect of the present invention, the recombinant protein of the second aspect of the present invention, the immunoconjugate of the sixth aspect of the present invention, or the immune cells of the eighth aspect of the present invention, or a combination thereof, for preparing a diagnostic reagent or kit, or for preparing a drug for treating diseases associated with high expression of GPRC5D.

In another preferred embodiment, the disease associated with high expression of GPRC5D is cancer, such as multiple myeloma.

In the eleventh aspect of the present invention, a non-diagnostic method for detecting GPRC5D protein in a sample *in vitro* is provided, comprising the following steps:
(s1) *in vitro,* contacting the sample with the antibody of the first aspect of the present invention;
(s2) detecting whether an antigen-antibody complex is formed, wherein the formation of the complex indicates the presence of GPRC5D protein in the sample.

In the twelfth aspect of the present invention, a method for preparing the antibody or its antigen-binding fragment of the first aspect of the present invention, or the recombinant protein of the second aspect of the present invention, is provided, comprising the following steps:
(si) cultivating the host cell of the fifth aspect of the present invention under conditions suitable for protein expression;
(sii) isolating the antibody or its antigen-binding fragment of the first aspect of the present invention, or the recombinant protein of the second aspect of the present invention from the cell culture.

In the thirteenth aspect of the present invention, a method for treating diseases associated with high expression of GPRC5D is provided, wherein the method comprises administering to a subject in need the antibody or its antigen-binding fragment of the first aspect of the present invention, the recombinant protein of the second aspect of the present invention, the immunoconjugate of the sixth aspect of the present invention, the chimeric antigen receptor of the seventh aspect of the present invention, the immune cells of the eighth aspect of the present invention, or the pharmaceutical composition of the ninth aspect of the present invention.

In another preferred embodiment, the disease associated with high expression of GPRC5D is cancer, such as multiple myeloma.

In another preferred embodiment, the subject in need is a human or a non-human mammal.

In the fourteenth aspect of the present invention, a bispecific antibody is provided, which comprises the antibody or its antigen-binding fragment of the first aspect of the present invention.

In another preferred embodiment, the bispecific antibody further comprises an antibody or its antigen-binding fragment targeting a second antigen, wherein the second antigen is selected from: CD3, CD16, CD32, 41BB.

In another preferred embodiment, the bispecific antibody comprises an antibody or its antigen-binding fragment targeting CD3.

In another preferred embodiment, the antibody or its antigen-binding fragment targeting CD3 comprises the heavy chain variable region as shown in SEQ ID NO: 97, and the light chain variable region as shown in SEQ ID NO: 98.

In another preferred embodiment, the bispecific antibody comprises three peptide chains:
Chain 1: GPRC5D scFv-Fc Hole
Chain 2: CD3 VH-CH1-Fc Knob
Chain 3: CD3 VL-CL; or
Chain 1: GPRC5D scFv-Fc Knob
Chain 2: CD3 VH-CH1-Fc Hole
Chain 3: CD3 VL-CL;
wherein the GPRC5D scFv is an scFv targeting GPRC5D, CD3 VH is the heavy chain variable region of an antibody targeting CD3, CD3 VL is the light chain variable region of an antibody targeting CD3, CH1 is the first constant region of the IgG heavy chain, and CL is the constant region of the light chain of the antibody.

In another preferred embodiment, the amino acid sequence of the Fc Knob is as shown in SEQ ID NO: 106, and the amino acid sequence of the Fc Knob is as shown in SEQ ID NO: 107.

In another preferred embodiment, the CL is the constant region of the human Kappa light chain.

In another preferred embodiment, the GPRC5D scFv is selected from the following group: SEQ ID NO: 53, 66, or 86.

In another preferred embodiment, the bispecific antibody comprises three peptide chains: Chain 1 as shown in any one of SEQ ID NOs: 102-104, Chain 2 as shown in SEQ ID NO: 101, and Chain 3 comprising the amino acid sequence as shown in SEQ ID NO: 98.

The present invention further provides a polynucleotide encoding the bispecific antibody of the fourteenth aspect of the present invention. It also provides a vector containing the said polynucleotide, and a host cell containing the said vector.

The present invention further provides an immunoconjugate and a pharmaceutical composition comprising the bispecific antibodyof the fourteenth aspect of the present invention.

The present invention also provides the use of the bispecific antibody of the fourteenth aspect of the present invention in the preparation of a pharmaceutical composition for treating diseases associated with high expression of GPRC5D, and a method for treating diseases associated with high expression of GPRC5D using the said bispecific antibody.

It should be understood that, within the scope of the present invention, the technical features described above and the technical features specifically described hereinafter (such as in the examples) can be combined with each other to form new or preferred technical solutions. Due to space limitations, these combinations will not be repeated here.

### Brief Description of Drawings

Figure 1 shows two structures of the humanized antibody: the native structure and the scFv-hFc structure.
Figure 2 shows the affinity of the native structure humanized antibody with K562-GPRC5D cells.
Figure 3 shows the affinity of the scFv-hFc structure humanized antibody with K562-GPRC5D (Figure 3a) and RPMI 8226 cells (Figure 3b).
Figure 4 shows the binding activity of the scFv-hFc structure humanized antibody with MM.1S (Figure 4a) and NCI-H929 (Figure 4b).
Figure 5 shows the vector insertion sequence structure of the GPRC5D CAR.
Figure 6 shows the self-activation and antigen-dependent activation of GPRC5D CAR Jurkat NFAT luc cells after co-culture with K562-GPRC5D (Figure 6a) or RPMI 8226 cells (Figure 6b).
Figure 7 shows the flow cytometry results of the transfection efficiency of GPRC5D CAR-T cells.
Figure 8 shows the concentration of inflammatory cytokines produced by GPRC5D CAR-T cells killing RPMI 8226 cells. Figure 8a shows IL-2 concentration, Figure 8b shows TNF-α concentration, and Figure 8c shows IFN-γ concentration.
Figure 9 shows the comparison of inflammatory cytokine concentrations after GPRC5D CAR-T cell co-culture with MM.1S or K562 cells and CAR-T cell culture alone. Figure 9a shows IL-2 concentration, Figure 9b shows TNF-α concentration, and Figure 9c shows IFN-γ concentration.
Figure 10 shows the cell proliferation flow cytometry results of GPRC5D CAR-T cells co-cultured with NCI-H929 or K562 cells, as well as CAR-T cells cultured alone.
Figure 11 shows the killing effect of GPRC5D CAR-T cells after co-culture with K562-luc (Figure 11a), K562-GPRC5D-luc (Figure 11b), NCI-H929-luc (Figure 11c), MM.1S-luc (Figure 11d), and RPMI 8226-luc (Figure 11e) target cells for 24 hours at different effector-to-target ratios.
Figure 12 shows the chemiluminescence detection results of B-NDG tumor-bearing mice treated with different dosing groups.
Figure 13 shows the endocytosis effects mediated by G05, G07, and G09 antibodies in K562-GPRC5D cells.
Figure 14 shows the *in vitro* binding activity of the GPRC5D&CD3 bispecific antibody; wherein Figure 14a shows the binding activity of the GPRC5D&CD3 bispecific antibody with H929 cells; Figure 14b shows the binding activity of the GPRC5D&CD3 bispecific antibody with Jurkat cells.
Figure 15 shows the *in vitro* killing efficiency of the GPRC5D&CD3 bispecific antibody; wherein Figure 15a shows the killing efficiency of the GPRC5D&CD3 bispecific antibody on the 8226 cell line after 48 hours; Figure 15b shows the killing efficiency on the H929 cell line after 48 hours; Figure 15c shows the killing efficiency on the MM.1S cell line after 48 hours; Figure 15d shows the killing efficiency on the K562 cell line after 48 hours.

### Detailed Description

Through extensive and in-depth research, as well as a large number of experimental screenings and humanization modifications, the inventors of the present invention obtained a group of humanized monoclonal antibodies with a novel amino acid sequence that specifically bind to human GPRC5D. Experimental results demonstrate that the humanized antibodies of the present invention exhibit strong affinity for the GPRC5D protein on the cell surface and possess excellent activity in stimulating T cell activation. CAR-T cells prepared based on the humanized antibody sequence of the present invention exhibit superior target cell-specific killing activity. The performance in all aspects is significantly superior to existing anti-GPRC5D antibodies. Based on these findings, the present invention was completed.

As used herein, the term "antibody" or "immunoglobulin" refers to a heterotetrameric protein of approximately 150,000 daltons with similar structural features, composed of two identical light chains (L) and two identical heavy chains (H). Each light chain is covalently linked to a heavy chain through a disulfide bond, and the number of disulfide bonds between the heavy chains of different immunoglobulin isotypes varies. Both the light and heavy chains also contain intra-chain disulfide bonds at regular intervals. One end of each heavy chain has a variable region (VH), followed by several constant regions. One end of each light chain has a variable region (VL), and the other end has a constant region. The constant region of the light chain is paired with the first constant region of the heavy chain, while the variable regions of the light and heavy chains are paired with each other. Specific amino acid residues form an interface between the variable regions of the light and heavy chains.

As used herein, the term "variable" refers to certain regions of the antibody's variable domain that vary in sequence, which contributes to the binding and specificity of various antibodies to their specific antigens. However, variability is not evenly distributed across the entire variable domain of the antibody. It is concentrated in three segments within the variable regions of the light and heavy chains, known as the Complementarity Determining Regions (CDRs) or hypervariable regions. The more conserved regions within the variable domain are referred to as the Framework Regions (FRs). The variable regions of natural heavy and light chains each contain four FR regions, which generally adopt a β-folded conformation, with three CDRs forming connecting loops that in some cases may form partial β-fold structures. The CDRs in each chain are tightly packed together through the FR regions and, along with the CDRs from the other chain, form the antigen-binding site of the antibody (see Kabat et al., NIH Publ. No. 91-3242, Vol. I, pp. 647-669 (1991)). The constant regions do not directly participate in antibody-antigen binding; however, they exhibit different effector functions, such as involvement in antibody-dependent cellular cytotoxicity.

The "light chain" of vertebrate antibodies (immunoglobulins) can be classified into two distinct types (referred to as κ and λ) based on the amino acid sequence of their constant regions. Immunoglobulins can be further categorized into different classes based on the amino acid sequence of their heavy chain constant regions. There are five main classes of immunoglobulins: IgA, IgD, IgE, IgG, and IgM, with some of them further subdivided into subclasses (isotypes), such as IgG1, IgG2, IgG3, IgG4, IgA1, and IgA2. The constant regions of the heavy chains corresponding to different immunoglobulin classes are designated as α, δ, ε, γ, and µ, respectively. The subunit structure and three-dimensional conformation of immunoglobulin classes are well known to those skilled in the art.

As used herein, the term "monoclonal antibody (mAb)" refers to an antibody obtained from a population that is essentially homogeneous, meaning that all antibodies within the population are identical, except for possible naturally occurring mutations. Monoclonal antibodies specifically target a single antigenic site. Unlike conventional polyclonal antibody preparations (which typically contain different antibodies targeting different epitopes), each monoclonal antibody is directed against a single epitope on the antigen. In addition to their specificity, the advantage of monoclonal antibodies is that they are not contaminated by other immunoglobulins. The modifier "monoclonal" refers to the characteristic of the antibody, which is derived from a homogeneous population of antibodies, and should not be interpreted to require any special method for antibody production.

The recombinant humanized monoclonal antibody targeting human GPRC5D provided by the present invention is selected from one or more of the following recombinant humanized monoclonal antibodies:
Monoclonal antibody named G01: The amino acid sequences of the heavy chain complementarity-determining regions (CDR1, CDR2, CDR3) are SEQ ID NO: 1, SEQ ID NO:2, SEQ ID NO:3, respectively; and the amino acid sequences of the light chain CDR1, CDR2, CDR3 are SEQ ID NO:4, SEQ ID NO:5, SEQ ID NO:6, respectively; the humanized heavy chain variable region amino acid sequence is SEQ ID NO:7; the humanized heavy chain variable region nucleic acid sequence is SEQ ID NO:8; the humanized light chain variable region amino acid sequence is SEQ ID NO:9; the humanized light chain variable region nucleic acid sequence is SEQ ID NO:10; the humanized scFv amino acid sequence is SEQ ID NO:11;
Monoclonal antibody named G02: The amino acid sequences of the heavy chain complementarity-determining regions (CDR1, CDR2, CDR3) are SEQ ID NO: 1, SEQ ID NO: 12, SEQ ID NO: 13, respectively; and the amino acid sequences of the light chain CDR1, CDR2, CDR3 are SEQ ID NO: 14, SEQ ID NO: 15, SEQ ID NO: 16, respectively; the humanized heavy chain variable region amino acid sequence is SEQ ID NO: 17; the humanized heavy chain variable region nucleic acid sequence is SEQ ID NO: 18; the humanized light chain variable region amino acid sequence is SEQ ID NO: 19; the humanized light chain variable region nucleic acid sequence is SEQ ID NO:20; the humanized scFv amino acid sequence is SEQ ID NO:21;
Monoclonal antibody named G03: The amino acid sequences of the heavy chain complementarity-determining regions (CDR1, CDR2, CDR3) are SEQ ID NO:22, SEQ ID NO:23, SEQ ID NO:24, respectively; and the amino acid sequences of the light chain CDR1, CDR2, CDR3 are SEQ ID NO:25, SEQ ID NO:26, SEQ ID NO:27, respectively; the humanized heavy chain variable region amino acid sequence is SEQ ID NO:28; the humanized heavy chain variable region nucleic acid sequence is SEQ ID NO:29; the humanized light chain variable region amino acid sequence is SEQ ID NO:30; the humanized light chain variable region nucleic acid sequence is SEQ ID NO:31; the humanized scFv amino acid sequence is SEQ ID NO:32;
Monoclonal antibody named G04: The amino acid sequences of the heavy chain complementarity-determining regions (CDR1, CDR2, CDR3) are SEQ ID NO:33, SEQ ID NO:34, SEQ ID NO:35, respectively; and the amino acid sequences of the light chain CDR1, CDR2, CDR3 are SEQ ID NO:36, SEQ ID NO:37, SEQ ID NO:38, respectively; the humanized heavy chain variable region amino acid sequence is SEQ ID NO:39; the humanized heavy chain variable region nucleic acid sequence is SEQ ID NO:40; the humanized light chain variable region amino acid sequence is SEQ ID NO:41; the humanized light chain variable region nucleic acid sequence is SEQ ID NO:42; the humanized scFv amino acid sequence is SEQ ID NO:43;
Monoclonal antibody named G05: The amino acid sequences of the heavy chain complementarity-determining regions (CDR1, CDR2, CDR3) are SEQ ID NO:44, SEQ ID NO:45, SEQ ID NO:46, respectively; and the amino acid sequences of the light chain CDR1, CDR2, CDR3 are SEQ ID NO:47, SEQ ID NO:37, SEQ ID NO:48, respectively; the humanized heavy chain variable region amino acid sequence is SEQ ID NO:49; the humanized heavy chain variable region nucleic acid sequence is SEQ ID NO:50; the humanized light chain variable region amino acid sequence is SEQ ID NO:51; the humanized light chain variable region nucleic acid sequence is SEQ ID NO:52; the humanized scFv amino acid sequence is SEQ ID NO:53;
Monoclonal antibody named G06: The amino acid sequences of the heavy chain complementarity-determining regions (CDR1, CDR2, CDR3) are SEQ ID NO:44, SEQ ID NO:54, SEQ ID NO:55, respectively; and the amino acid sequences of the light chain CDR1, CDR2, CDR3 are SEQ ID NO:47, SEQ ID NO:37, SEQ ID NO:48, respectively; the humanized heavy chain variable region amino acid sequence is SEQ ID NO:56; the humanized heavy chain variable region nucleic acid sequence is SEQ ID NO:57; the humanized light chain variable region amino acid sequence is SEQ ID NO:51; the humanized light chain variable region nucleic acid sequence is SEQ ID NO:52; the humanized scFv amino acid sequence is SEQ ID NO:58;
Monoclonal antibody named G07: The amino acid sequences of the heavy chain complementarity-determining regions (CDR1, CDR2, CDR3) are SEQ ID NO:44, SEQ ID NO:59, SEQ ID NO:60, respectively; and the amino acid sequences of the light chain CDR1, CDR2, CDR3 are SEQ ID NO:61, SEQ ID NO:37, SEQ ID NO:48, respectively; the humanized heavy chain variable region amino acid sequence is SEQ ID NO:62; the humanized heavy chain variable region nucleic acid sequence is SEQ ID NO:63; the humanized light chain variable region amino acid sequence is SEQ ID NO:64; the humanized light chain variable region nucleic acid sequence is SEQ ID NO:65; the humanized scFv amino acid sequence is SEQ ID NO:66;
Monoclonal antibody named G08: The amino acid sequences of the heavy chain complementarity-determining regions (CDR1, CDR2, CDR3) are SEQ ID NO:67, SEQ ID NO:68, SEQ ID NO:69, respectively; and the amino acid sequences of the light chain CDR1, CDR2, CDR3 are SEQ ID NO:70, SEQ ID NO:71, SEQ ID NO:72, respectively; the humanized heavy chain variable region amino acid sequence is SEQ ID NO:73; the humanized heavy chain variable region nucleic acid sequence is SEQ ID NO:74; the humanized light chain variable region amino acid sequence is SEQ ID NO:75; the humanized light chain variable region nucleic acid sequence is SEQ ID NO:76; the humanized scFv amino acid sequence is SEQ ID NO:77;
Monoclonal antibody named G09: The amino acid sequences of the heavy chain complementarity-determining regions (CDR1, CDR2, CDR3) are SEQ ID NO:78, SEQ ID NO:79, SEQ ID NO:60, respectively; and the amino acid sequences of the light chain CDR1, CDR2, CDR3 are SEQ ID NO:80, SEQ ID NO:37, SEQ ID NO:81, respectively; the humanized heavy chain variable region amino acid sequence is SEQ ID NO:82; the humanized heavy chain variable region nucleic acid sequence is SEQ ID NO:83; the humanized light chain variable region amino acid sequence is SEQ ID NO:84; the humanized light chain variable region nucleic acid sequence is SEQ ID NO:85; the humanized scFv amino acid sequence is SEQ ID NO:86;
Monoclonal antibody named G10: The amino acid sequences of the heavy chain complementarity-determining regions (CDR1, CDR2, CDR3) are SEQ ID NO:87, SEQ ID NO:88, SEQ ID NO:89, respectively; and the amino acid sequences of the light chain CDR1, CDR2, CDR3 are SEQ ID NO:90, SEQ ID NO:26, SEQ ID NO:91, respectively; the humanized heavy chain variable region amino acid sequence is SEQ ID NO:92; the humanized heavy chain variable region nucleic acid sequence is SEQ ID NO:93; the humanized light chain variable region amino acid sequence is SEQ ID NO:94; the humanized light chain variable region nucleic acid sequence is SEQ ID NO:95; the humanized scFv amino acid sequence is SEQ ID NO:96.

Based on the above technical solution, the constant region of the light chain of the recombinant humanized monoclonal antibody is of the κ chain type, and the constant region of the heavy chain is of the IgG type.

The present invention not only includes full monoclonal antibodies, but also includes antibody fragments with immunological activity, such as Fab or (Fab")2 fragments; scFv; antibody heavy chains; and antibody light chains.

The present invention also provides other proteins or fusion expression products that contain the antibody of the present invention. Specifically, the invention includes any protein or protein conjugate and fusion expression product that contains heavy and light chains with variable regions, as long as the variable regions have the same or at least about 90% homology to the variable regions of the heavy and light chains of the antibody of the present invention, preferably at least about 95% homology.

The variable regions of the heavy chain and/or light chain of the antibody of the present invention are of particular interest because they are at least partially involved in antigen binding. Therefore, the present invention includes molecules with monoclonal antibody light and heavy chain variable regions that carry CDRs, as long as the CDRs have more than 90% (preferably more than 95%, most preferably more than 98%) homology with the CDRs identified herein.

As used herein, the terms "fragment," "derivative," and "analogue" refer to polypeptides that essentially retain the same biological function or activity as the antibody of the present invention. The polypeptide fragments, derivatives, or analogues of the present invention may be (i) polypeptides in which one or more conservative or non-conservative amino acid residues (preferably conservative amino acid residues) are substituted, where such substituted amino acid residues may or may not be encoded by the genetic code, or (ii) polypeptides having substituent groups on one or more amino acid residues, or (iii) polypeptides formed by fusing a mature polypeptide with another compound (such as a compound that extends the half-life of the polypeptide, e.g., polyethylene glycol), or (iv) polypeptides formed by fusing an additional amino acid sequence to the polypeptide sequence (such as a leader sequence, a secretion sequence, a sequence for purifying the polypeptide, or a protein sequence, or a fusion protein with a 6His tag). According to the teachings of the present invention, these fragments, derivatives, and analogues fall within the scope known to those skilled in the art.

The antibodies of the present invention refer to polypeptides that possess binding activity to the human GPRC5D protein and include the aforementioned CDR regions. This term also encompasses variant forms of polypeptides that contain the above CDR regions and exhibit the same function as the antibodies of the present invention. These variants include (but are not limited to) one or more (typically 1-50, preferably 1-30, more preferably 1-20, most preferably 1-10) amino acid deletions, insertions, and/or substitutions, as well as the addition of one or more (typically no more than 20, preferably no more than 10, more preferably no more than 5) amino acids at the C-terminus and/or N-terminus. For example, in the art, substituting with amino acids that have similar or equivalent properties typically does not alter the function of the protein. Similarly, adding one or more amino acids at the C-terminus and/or N-terminus generally does not affect the protein's function. This term also includes active fragments and active derivatives of the antibodies of the present invention.

The variant forms of the polypeptide include: homologous sequences, conservative variants, allelic variants, natural mutants, induced mutants, proteins encoded by DNA that can hybridize with the DNA encoding the antibody of the present invention under high or low stringency conditions, and polypeptides or proteins obtained using antisera raised against the antibody of the present invention.

The present invention also provides other polypeptides, such as fusion proteins containing human antibodies or fragments thereof. In addition to nearly full-length polypeptides, the present invention also includes fragments of the antibodies of the present invention. Typically, these fragments contain at least about 50 consecutive amino acids of the antibody of the present invention, preferably at least about 60 consecutive amino acids, more preferably at least about 80 consecutive amino acids, and most preferably at least about 100 consecutive amino acids.

In the present invention, "conservative variants of the antibody of the present invention" refers to polypeptides that differ from the amino acid sequence of the antibody of the present invention by no more than 10, preferably no more than 8, more preferably no more than 5, and most preferably no more than 3 amino acids, where the substitutions are made with amino acids having similar or close properties. These conservative variant polypeptides are preferably generated by amino acid substitutions as described in Table 1.

**Table 1**

| Original Residue | Representative Substitutes | Preferred Substitutes |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The invention also provides polynucleotide molecules encoding the aforementioned antibodies, fragments thereof, or fusion proteins. The polynucleotides of the invention can be in the form of DNA or RNA. The DNA form includes cDNA, genomic DNA, or artificially synthesized DNA. The DNA can be single-stranded or double-stranded. It can be either a coding strand or a non-coding strand. The coding sequence for the mature polypeptide may be identical to or a degenerate variant of, for example, the coding sequence shown in SEQ ID NO: 50 or 52. As used herein, "degenerate variant" refers to a nucleic acid sequence that encodes the same amino acid sequence as the polypeptides of the present invention but differs in sequence from the coding sequence shown in, for example, SEQ ID NO: 50 or 52.

Polynucleotides encoding the mature polypeptides of the present invention include: Sequences encoding only the mature polypeptide; Sequences encoding the mature polypeptide along with various additional coding sequences; Sequences encoding the mature polypeptide (and optionally additional coding sequences) along with non-coding sequences.

The term "polynucleotide encoding a polypeptide" refers to a polynucleotide that includes the nucleic acid sequence encoding the polypeptide, and may also include additional coding and/or non-coding sequences.

The invention further relates to polynucleotides that hybridize with the sequences described above and exhibit at least 50%, preferably at least 70%, more preferably at least 80% sequence identity between the two sequences. The invention specifically relates to polynucleotides that can hybridize under stringent conditions with the polynucleotides described in the invention. As used herein, "stringent conditions" refers to: (1) hybridization and wash conditions at low ionic strength and high temperature, such as 0.2×SSC, 0.1% SDS, 60°C; (2) hybridization in the presence of a denaturant, such as 50% (v/v) formamide, 0.1% bovine serum albumin (BSA)/0.1% Ficoll at 42°C; or (3) hybridization occurring only when the sequence identity between the two sequences is at least 90%, more preferably at least 95%. Moreover, the polynucleotides that can hybridize encode polypeptides that exhibit the same biological function and activity as the mature polypeptides shown in, for example, SEQ ID NO: 49 or SEQ ID NO: 51.

The full-length nucleotide sequence of the antibody of the present invention, or its fragments, can typically be obtained using PCR amplification, recombinant methods, or chemical synthesis methods. One feasible approach is to synthesize the relevant sequence, particularly when the fragment length is shorter. Generally, longer fragments are synthesized by first generating several small segments and then linking them together. Additionally, the coding sequence for the heavy chain can be fused with an expression tag (such as 6His), forming a fusion protein.

Once the relevant sequence is obtained, recombinant methods can be used to produce large quantities of the sequence. This typically involves cloning the sequence into a vector, then transfecting it into cells, and subsequently isolating the sequence from the proliferating host cells using conventional methods. The biomolecules (nucleic acids, proteins, etc.) involved in the present invention include biomolecules that exist in isolated forms.

Currently, the DNA sequence encoding the protein of the present invention (or its fragments or derivatives) can be fully obtained through chemical synthesis. This DNA sequence can then be introduced into various known existing DNA molecules (or vectors) and cells commonly used in the field. Additionally, mutations can be introduced into the protein sequence of the present invention through chemical synthesis.

The present invention also relates to vectors that include the appropriate DNA sequence mentioned above, along with suitable promoters or control sequences. These vectors can be used to transform appropriate host cells to enable them to express the protein.

Host cells can be prokaryotic cells, such as bacterial cells; lower eukaryotic cells, such as yeast cells; or higher eukaryotic cells, such as mammalian cells. Representative examples include: *Escherichia coli, Streptomyces* species; bacterial cells of *Salmonella typhimurium;* fungal cells such as yeast; insect cells such as *Drosophila* S2 or Sf9 cells; and animal cells such as CHO, COS7, or 293 cells.

Recombinant DNA transformation of host cells can be performed using conventional techniques well known to those skilled in the art. When the host is a prokaryote such as *Escherichia coli,* competent cells capable of taking up DNA can be harvested after the exponential growth phase, treated with CaCl2, a widely known method in the field. Another method involves using MgCl2. If necessary, transformation can also be carried out using electroporation. When the host is a eukaryote, several DNA transfection methods may be employed, including calcium phosphate co-precipitation, conventional mechanical methods such as microinjection, electroporation, liposome packaging, and others.

The resulting transformants can be cultured using conventional methods to express the polypeptides encoded by the genes of the present invention. Depending on the host cell used, various standard culture media may be chosen. Culturing is performed under conditions suitable for host cell growth. Once the host cells reach an appropriate density, expression of the gene can be induced by appropriate methods (such as temperature shifts or chemical induction) to activate the selected promoter, followed by further culturing.

The recombinant polypeptides produced in the above methods can be expressed intracellularly, on the cell membrane, or secreted into the extracellular space. If required, the recombinant protein can be isolated and purified using various separation methods based on its physical, chemical, and other properties. These methods are well known to those skilled in the art. Examples of such methods include but are not limited to: conventional refolding procedures, treatment with protein precipitants (salt precipitation), centrifugation, osmolysis, ultrafiltration, ultracentrifugation, size-exclusion chromatography (gel filtration), adsorption chromatography, ion-exchange chromatography, high-performance liquid chromatography (HPLC), and other various liquid chromatography techniques, as well as combinations of these methods.

The antibodies of the present invention can be used alone or in combination with detectable markers (for diagnostic purposes), therapeutic agents, PK (protein kinase) modification parts, or any combination or conjugation of the above substances.

Detectable markers for diagnostic purposes include, but are not limited to: fluorescent or luminescent markers, radioactive markers, MRI (magnetic resonance imaging) or CT (computed tomography) contrast agents, or enzymes that can produce detectable products.

Therapeutic agents that can be conjugated include, but are not limited to: insulin, IL-2, interferon, calcitonin, GHRH peptides, gut peptide analogs, albumin, antibody fragments, cytokines, and hormones.

Additionally, therapeutic agents that can be conjugated or linked to the antibodies of the present invention include, but are not limited to: 1. Radioactive isotopes; 2. Biotoxins; 3. Cytokines such as IL-2, etc.; 4. Gold nanoparticles/nanorods; 5. Viral particles; 6. Liposomes; 7. Nanomagnetic particles; 8. Prodrug-activating enzymes (e.g., DT myocardial enzyme (DTD) or biphenyl hydrolase-like protein (BPHL)); 9. Chemotherapeutic agents (e.g., cisplatin) or any form of nanoparticles, etc.

The present invention also provides a chimeric antigen receptor (CAR), which includes an extracellular domain, a transmembrane domain, and an intracellular domain. The extracellular domain includes a target-specific binding element (also referred to as the antigen-binding domain). The intracellular domain includes a co-stimulatory signaling region and a ζ chain portion. The co-stimulatory signaling region refers to a portion of the intracellular domain of a co-stimulatory molecule, which is a cell surface molecule required for an effective lymphocyte response to an antigen, distinct from the antigen receptor or their ligands.

A linker can be incorporated between the extracellular domain and the transmembrane domain, or between the intracellular domain and the transmembrane domain of the CAR. As used herein, the term "linker" generally refers to any oligopeptide or polypeptide that serves to connect the transmembrane domain to the extracellular domain or intracellular domain of the polypeptide chain. The linker can comprise 0 to 300 amino acids, preferably 2 to 100 amino acids, and most preferably 3 to 50 amino acids.

In one preferred embodiment of the present invention, the extracellular domain of the CAR provided by the present invention includes an antigen-binding domain that targets human GPRC5D. When expressed in T cells, the CAR of the present invention is capable of antigen recognition based on antigen binding specificity. Upon binding to its associated antigen, it affects tumor cells, resulting in tumor cell growth inhibition, induction of cell death, or other effects, leading to a reduction or elimination of the tumor burden in the patient. The antigen-binding domain is preferably fused with the intracellular domains from one or more co-stimulatory molecules and the ζ chain. More preferably, the antigen-binding domain is fused with the intracellular domains composed of the 4-1BB signaling domain and the CD3ζ signaling domain.

As used herein, the terms "antigen-binding domain" and "single-chain antibody fragment" refer to Fab fragments, Fab' fragments, F(ab')2 fragments, or single Fv fragments that possess antigen-binding activity. Fv antibodies contain the variable regions of the antibody heavy chain and light chain but lack the constant region, representing the smallest antibody fragment with all antigen-binding sites. Generally, Fv antibodies also include a peptide linker between the VH and VL domains and are capable of forming the structure required for antigen binding. The antigen-binding domain is typically a scFv (single-chain variable fragment). The size of an scFv is typically 1/6 of a complete antibody. The single-chain antibody is preferably an amino acid chain sequence encoded by a single nucleotide chain. As a preferred embodiment of the present invention, the antigen-binding domain comprises an antibody that specifically recognizes human GPRC5D, more preferably a single-chain antibody.

For the scFv and transmembrane regions (transmembrane domain), the CAR can be designed to include a transmembrane domain fused to the extracellular domain of the CAR. In one embodiment, the transmembrane domain naturally associated with one of the domains in the CAR is used. In some examples, a transmembrane domain can be selected or modified through amino acid substitutions to prevent such domains from associating with the transmembrane domains of the same or different surface membrane proteins, thereby minimizing interactions with other members of the receptor complex.

In a preferred embodiment of the present invention, the CAR-T cells constructed using the humanized anti-GPRC5D scFv of the present invention can further enhance their cytotoxic effects and tumor-clearing ability.

The present invention also provides a pharmaceutical composition, which contains the aforementioned antibody or its active fragment, or fusion protein, or immune cells expressing the antibody, along with a pharmaceutically acceptable carrier. Generally, these substances can be formulated in a non-toxic, inert, and pharmaceutically acceptable aqueous carrier medium, where the pH is typically around 5-8, preferably around 6-8, although the pH may vary depending on the nature of the formulated substances and the condition being treated. The prepared pharmaceutical composition can be administered by conventional methods, including (but not limited to): oral, respiratory, intratumoral, intraperitoneal, intravenous, or local administration.

The pharmaceutical composition of the present invention contains a safe and effective amount (e.g., 0.001-99 wt%, preferably 0.01-90 wt%, more preferably 0.1-80 wt%) of the monoclonal antibody (or its conjugate) of the present invention, along with a pharmaceutically acceptable carrier or excipient. Such carriers include (but are not limited to): saline, buffer solutions, glucose, water, glycerol, ethanol, and combinations thereof. The pharmaceutical formulation should be matched to the administration route. The pharmaceutical composition of the present invention can be formulated into an injectable form, for example, prepared using saline or an aqueous solution containing glucose and other excipients through conventional methods. The pharmaceutical composition, such as injectable solutions, should be manufactured under sterile conditions. The amount of active ingredient to be administered is a therapeutically effective amount, such as about 1 microgram/kg body weight to about 10 milligrams/kg body weight per day. Additionally, the antibody of the present invention may also be used in combination with other therapeutic agents.

When using the pharmaceutical composition, a safe and effective amount of the immunoconjugate is administered to a mammal, where this safe and effective amount is typically at least about 10 micrograms/kg body weight, and in most cases does not exceed about 8 milligrams/kg body weight, preferably the dosage is about 10 micrograms/kg body weight to about 1 milligram/kg body weight. Of course, the specific dosage should also consider factors such as the administration route, the patient's health condition, and other factors, all of which are within the skill of the treating physician.

The present invention also provides the use of the antibody or its antigen-binding fragments of the present invention, the chimeric antigen receptor (CAR), and engineered immune cells expressing said chimeric antigen receptor, or the pharmaceutical conjugates or pharmaceutical compositions of the present invention, in the prevention and/or treatment of cancers containing tumor cells expressing GPRC5D (such as multiple myeloma).

Compared to the prior art, the present invention has the following advantages and beneficial effects:
The present invention utilizes B-cell cloning technology to provide a recombinant humanized monoclonal antibody targeting human GPRC5D. Importantly, the specificity of the recombinant humanized monoclonal antibody targeting human GPRC5D has a high affinity and good specificity, which can address the gaps in the market related to the diagnostic and therapeutic applications of recombinant humanized monoclonal antibodies targeting human GPRC5D. In addition, the GPRC5D antibody sequence can be constructed into CAR-T cells, bispecific antibodies, or antibody-drug conjugates, which can be used for the treatment of subsequent tumor diseases.

The present invention is further illustrated with specific embodiments. It should be understood that these embodiments are for illustrative purposes only and are not intended to limit the scope of the invention. Experimental methods in the following embodiments that do not specify particular conditions are generally conducted under conventional conditions, such as those described in Sambrook et al., Molecular Cloning: A Laboratory Manual (New York: Cold Spring Harbor Laboratory Press, 1989), or according to conditions recommended by the manufacturer. Unless otherwise specified, percentages and parts are calculated by weight. The experimental materials and reagents used in the following examples are commercially available unless otherwise stated.

### Example 1: Affinity Flow Cytometry Detection of Native Structure Humanized Antibody

The anti-GPRC5D antibody (MCARH109) developed by Eureka Company was set as the control antibody G00. Its scFv sequence comes from Eureka's patent (CN107428829B). According to experimental needs, the control antibody was constructed into humanized antibody forms, such as scFv-hFc antibody, CAR-T, CAR-jurkat-Nfat-Luc cells, etc.

To obtain a human recombinant monoclonal antibody targeting the human GPRC5D protein, the present invention constructs a recombinant protein expressing the N-terminal extracellular domain of human GPRC5D in mammalian cells as an immunogen. Immunization was carried out using New Zealand white rabbits. After immunization, antigen-coated magnetic beads were used to select B lymphocytes. After expanding the single B lymphocytes, flow cytometry was used to detect the expression of GPRC5D antibodies. Positive clones were subjected to gene sequencing, and the gene sequence of the rabbit monoclonal antibody was obtained. The rabbit monoclonal antibody was then humanized through a rabbit-human chimerization process, yielding 10 humanized GPRC5D monoclonal antibodies, which were named G01, G02, G03, G04, G05, G06, G07, G08, G09, and G10.

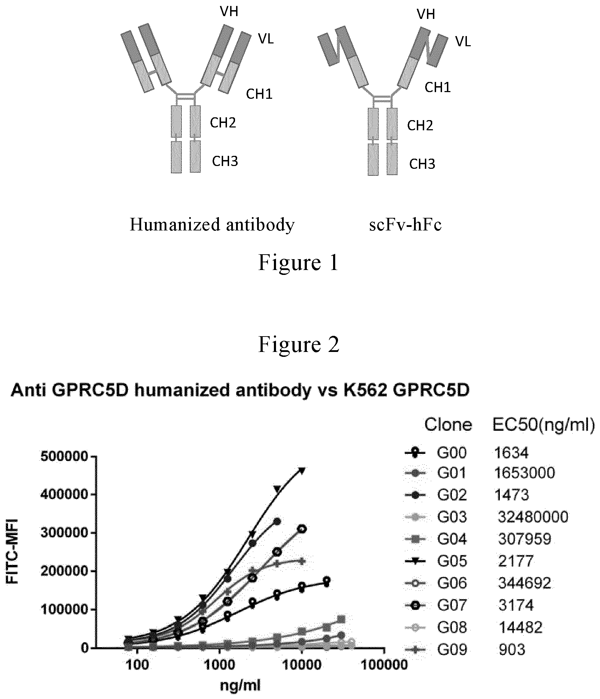

The antibodies G00 to G09 were constructed into humanized IgG1-type antibody vector plasmids. The recombinant vector plasmids were transfected into 293 cells, and the supernatant was harvested to purify and concentrate the antibodies (structure shown in Figure 1, left image). After quantification, the antibodies were used for flow cytometry detection experiments.

Flow Cytometry Detection was carried out according to the following steps:
(1) Dilution of humanized antibodies G00~G09 to equal concentrations: 40 µg/ml, 20 µg/ml, 10 µg/ml, 5 µg/ml, 2.5 µg/ml, 1.25 µg/ml, 0.625 µg/ml, 0.375 µg/ml, and 0 µg/ml.
(2) Preparation of the K562-GPRC5D stable transfected cell line (K562 cells stably expressing GPRC5D): After washing with PBS, discard the supernatant, resuspend the cells in PBS, and perform a cell count. Adjust the cell density to 3 * 10^6 cells/ml. Add 50 µl of the cell suspension per well into a labeled 96-well plate.
(3) Incubate the diluted humanized antibody solutions of each concentration in the 96-well plate for 30 minutes.
(4) Wash the wells twice with PBS, centrifuge to remove the supernatant.
(5) Add 50 µl of secondary antibody to each well and incubate for 30 minutes. After washing the plate again, resuspend the cells in PBS and analyze the plate using a flow cytometer.

As shown in Figure 2, G02, G05, G07, and G09 exhibit good binding activity to K562-GPRC5D, which is superior to the control antibody G00.

### Example 2: Affinity Flow Cytometry Detection of scFv-hFc Humanized Antibody

The G00~G09 antibodies were constructed into scFv-hFc humanized IgG1 format plasmids. The recombinant plasmids were transfected into 293 cells, and the supernatants were harvested and purified to concentrate the antibodies (structure shown in Figure 1, right). After quantification, the antibodies were used in flow cytometry experiments.

Flow cytometry detection was performed as follows:
(1) Dilute G00~G09 scFv-hFc IgG1 humanized antibodies to equal concentrations: 40 µg/ml, 20 µg/ml, 10 µg/ml, 5 µg/ml, 2.5 µg/ml, 1.25 µg/ml, 0.625 µg/ml, 0.375 µg/ml, and 0 µg/ml.
(2) Take the cultured K562-GPRC5D stable cell line and RPMI 8226 cells (natural multiple myeloma cells that express GPRC5D), wash once with PBS, remove the supernatant, and resuspend the cells in PBS. Adjust the cell density to 3 * 10^6 cells/ml, and add 50 µl of the cell suspension per well to a labeled 96-well plate.
(3) Add the diluted antibody solutions at various concentrations to the 96-well plate and incubate for 30 minutes.
(4) Wash the plate twice with PBS, centrifuge to remove the supernatant.
(5) Add 50 µl of secondary antibody and incubate for 30 minutes. After washing the plate again, resuspend the cells in PBS, and analyze using a flow cytometer.

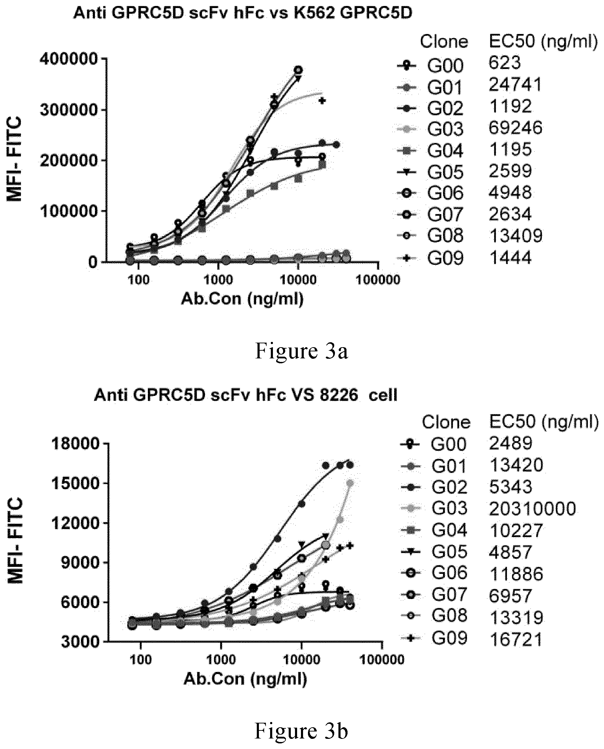

As shown in Figure 3, G02, G05, G07, and G09 exhibit good affinity for both K562-GPRC5D (Figure 3a) and 8226 cells (Figure 3b), superior to the control antibody G00.

### Example 3: Affinity Flow Cytometry Detection of scFv-hFc Humanized Antibody Binding to MM.1S and H929 Cells

The binding activity of the scFv-hFc humanized antibodies prepared in Example 2 was tested on MM.1S and NCI-H929 cells (both MM.1S and NCI-H929 are natural multiple myeloma cell lines that express GPRC5D). The following steps were performed:
(1) Dilute G00~G09 scFv-hFc IgG1 humanized antibodies to a concentration of 20 µg/ml.
(2) Take the cultured MM.1S cells and NCI-H929 cells, wash once with PBS, remove the supernatant, and resuspend the cells in PBS. Adjust the cell density to 3 * 10^6 cells/ml, and add 50 µl of the cell suspension per well to a labeled 96-well plate.
(3) Add the diluted antibody solutions to the 96-well plate and incubate for 30 minutes.
(4) Wash the plate twice with PBS, centrifuge to remove the supernatant.
(5) Add 50 µl of secondary antibody and incubate for 30 minutes. After washing the plate again, resuspend the cells in PBS, and analyze using a flow cytometer.

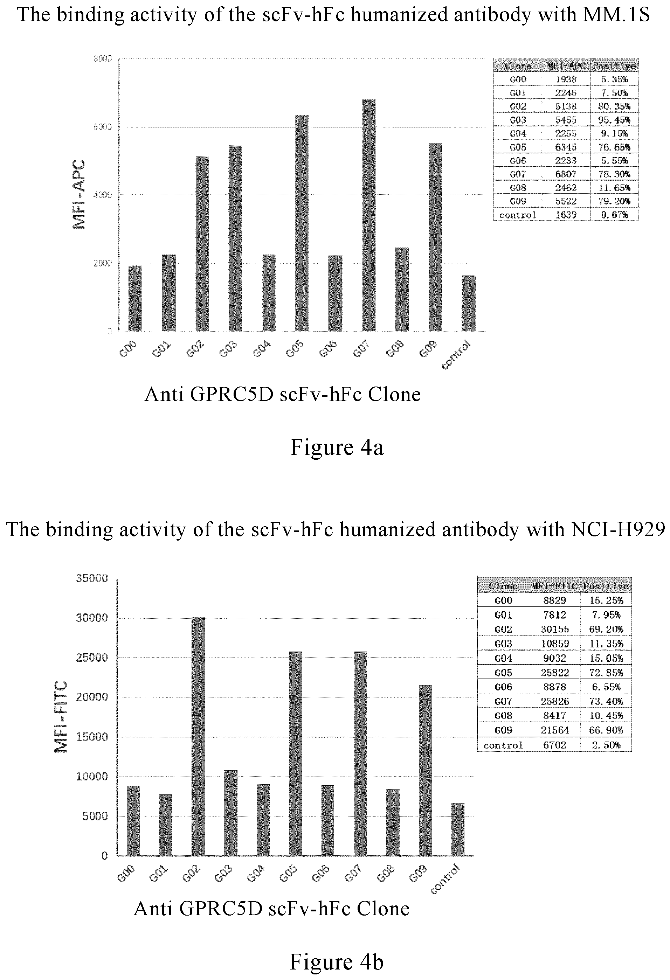

As shown in Figure 4, G02, G05, G07, and G09 exhibit good binding activity to MM.1S cells (Figure 4a) and NCI-H929 cells (Figure 4b), significantly outperforming the control antibody G00.

### Example 4: CAR-Jurkat-NFAT-Luc Cells Autostimulation and Antigen-Dependent Activation Detection

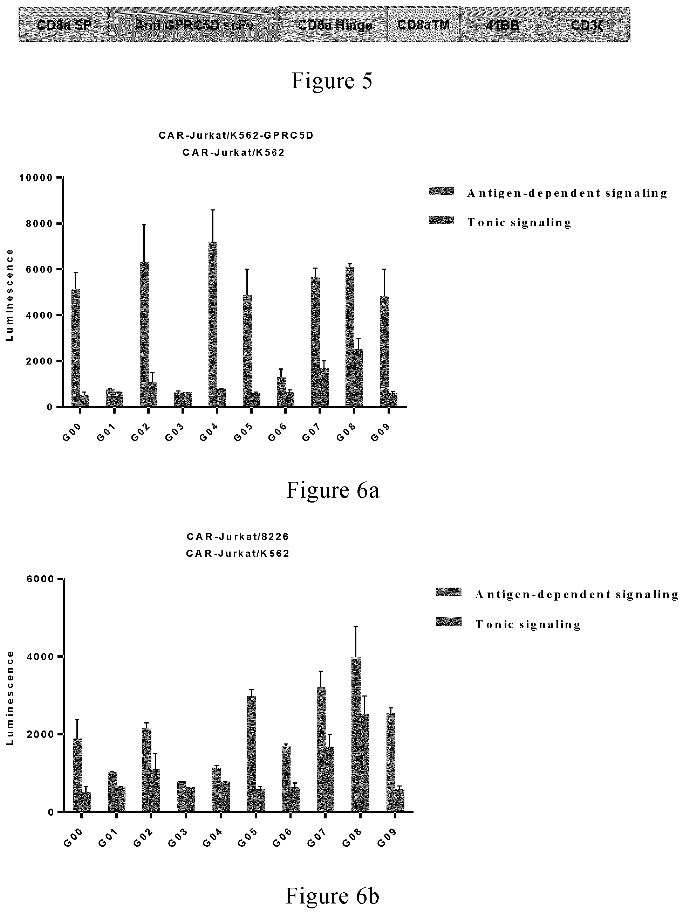

As shown in Figure 5, the scFv sequences of G00~G09 were individually constructed into CAR lentiviral vectors. The vectors were used for lentivirus packaging, and the virus-containing supernatant was concentrated and purified. This was then used to infect Jurkat-NFAT-Luc cells, generating infected cells, namely CAR-Jurkat-NFAT-Luc cells. These CAR-Jurkat-NFAT-Luc cells were co-cultured with K562, K562-GPRC5D, and RPMI 8226 cells for 48 hours, and bioluminescence values were detected.

As shown in Figure 6, Figure 6a displays the results of CAR-Jurkat-NFAT-Luc cells co-cultured with K562-GPRC5D or K562 cells, while Figure 6b shows the results of CAR-Jurkat-NFAT-Luc cells co-cultured with RPMI 8226 or K562 cells. It can be observed that the CAR-Jurkat-NFAT-Luc cells exhibit a low background of autostimulation and a high antigen-dependent activation signal, particularly in CAR-Jurkat-NFAT-Luc cells expressing scFvs G02, G05, G07, and G09.

### Example 5: CAR-T Cells Preparation

30 mL of human peripheral blood was collected into a heparinized blood collection tube. The blood was centrifuged at 600g for 10 minutes, and the upper yellow plasma was carefully collected and inactivated for later use. The remaining blood components were mixed with PBS and layered on top of a lymphocyte separation medium in a centrifuge tube. The mixture was centrifuged at 1000g for 10 minutes. The white buffy coat layer cells were carefully collected, washed with PBS, and centrifuged to remove the supernatant. The cell pellet was resuspended in PBS for counting. After centrifuging to remove the supernatant, the cells were resuspended in sorting buffer. Based on the cell count, anti-human CD3 nanomagnetic beads and the Medtronic magnetic bead sorting system were used to isolate the CD3-positive cell population. After counting the isolated CD3-positive cells, they were activated using CD3/CD28 magnetic beads for 24 hours. Following activation, the cells were infected with G00~G09 CAR lentivirus. After overnight infection, the cells were washed, and CAR expression efficiency was assessed by flow cytometry after 48 hours.

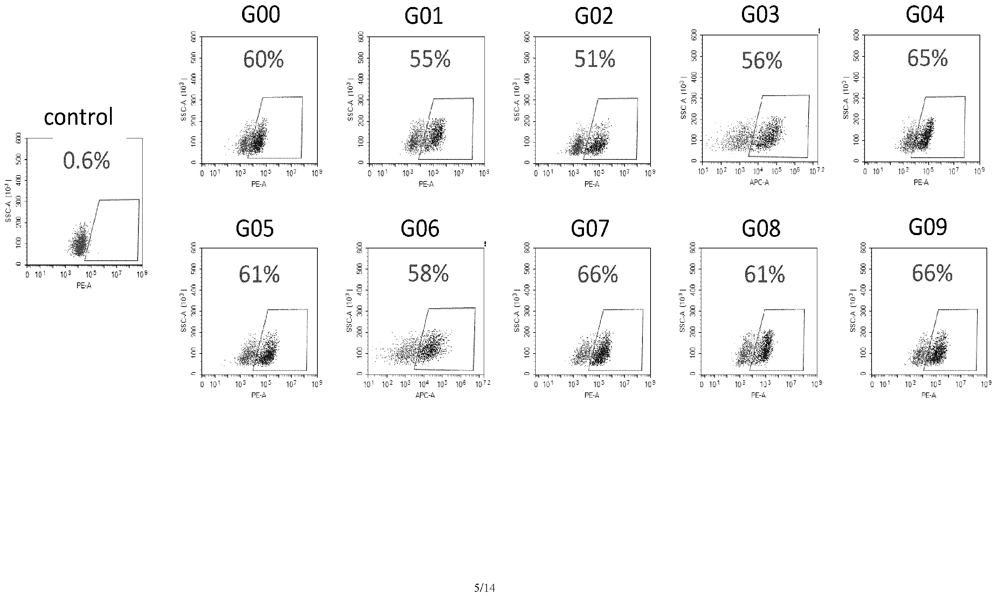

As shown in Figure 7, the G00~G09 CAR-T cells all demonstrated high transfection efficiency.

### Example 6: CAR-T Cell Cytotoxicity Assay and Cytokine Detection in RPMI-8226 Cells

The G00~G09 CAR-T cells and T cells prepared in Example 5 were washed with PBS and resuspended in culture medium for counting. The CAR-T cell concentration and T cell concentration were adjusted to 1*10^5 cells/mL. The CAR-T cells and T cells were then added to a 96-well plate, with 100 µL of cell suspension per well.

The RPMI-8226 cells in culture were washed with PBS, resuspended in culture medium, and counted. The cell concentration was adjusted to 1*10^5 cells/mL, and 100 µL of RPMI-8226 cell suspension was added to the corresponding wells of the 96-well plate.

The CAR-T cell and RPMI-8226 cell suspensions were mixed well and incubated overnight in a CO₂ incubator for 24 hours. After incubation, the 96-well plate was centrifuged at 300g for 10 minutes, and 150 µL of the supernatant was transferred to a new 96-well plate. The concentrations of IL-2, TNF-α, and IFN-γ in the supernatant were measured using ELISA kits.

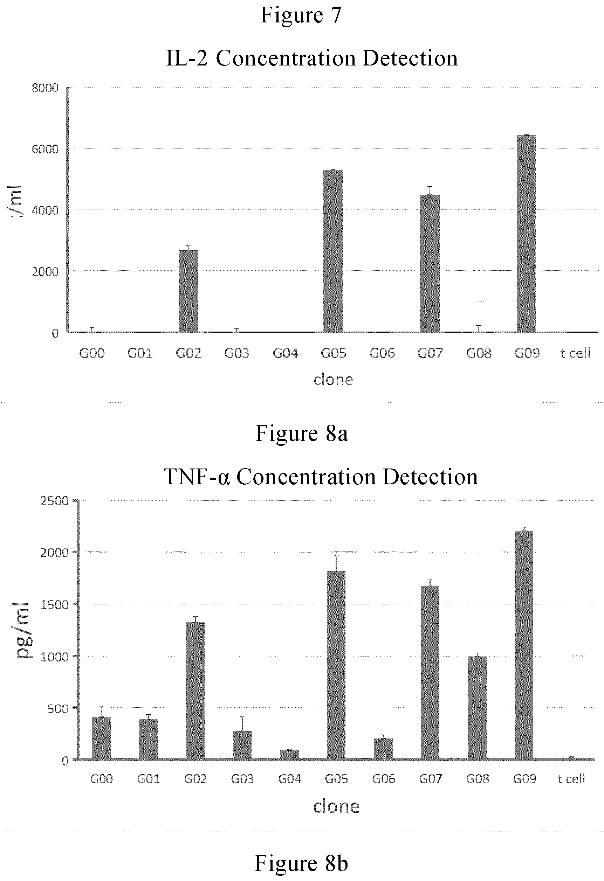

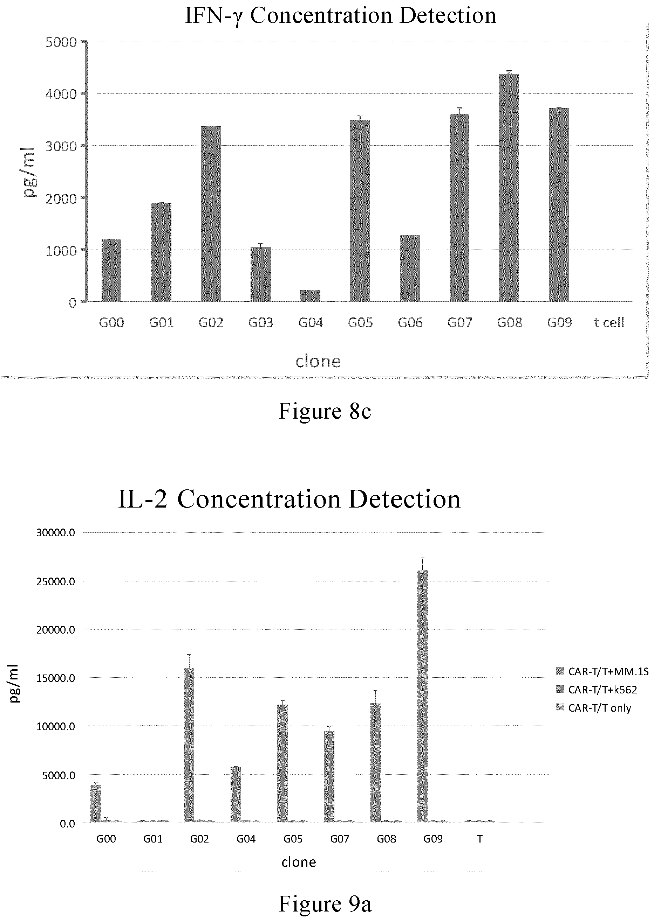

As shown in Figure 8, the concentrations of IL-2 (Figure 8a), TNF-α (Figure 8b), and IFN-γ (Figure 8c) in the supernatant were detected. It can be seen that G02, G05, G07, and G09 CAR-T cells specifically killed RPMI-8226 cells and induced higher levels of inflammatory cytokines.

### Example 7: CAR-T Cell Cytotoxicity Assay and Cytokine Detection in MM.1S and K562 Cells

The G00~G09 CAR-T cells and T cells prepared in Example 5 were washed with PBS and resuspended in culture medium for counting. The CAR-T cell concentration and T cell concentration were adjusted to 1*10^5 cells/mL. The CAR-T cells and T cells were added to a 96-well plate, with 100 µL of cell suspension per well.

MM.1S and K562 cells in culture were washed with PBS, resuspended in culture medium, and counted. The cell concentration was adjusted to 1*10^5 cells/mL, and 100 µL of MM. 1S and K562 cell suspensions were added to the corresponding wells of the 96-well plate.

The CAR-T cells or T cells were mixed with the MM.1S or K562 cells and incubated overnight in a CO₂ incubator for 24 hours. After incubation, the 96-well plate was centrifuged at 300g for 10 minutes, and 150 µL of the culture supernatant was transferred to a new 96-well plate. The concentrations of IL-2, TNF-α, and IFN-γ in the supernatant were measured using ELISA kits.

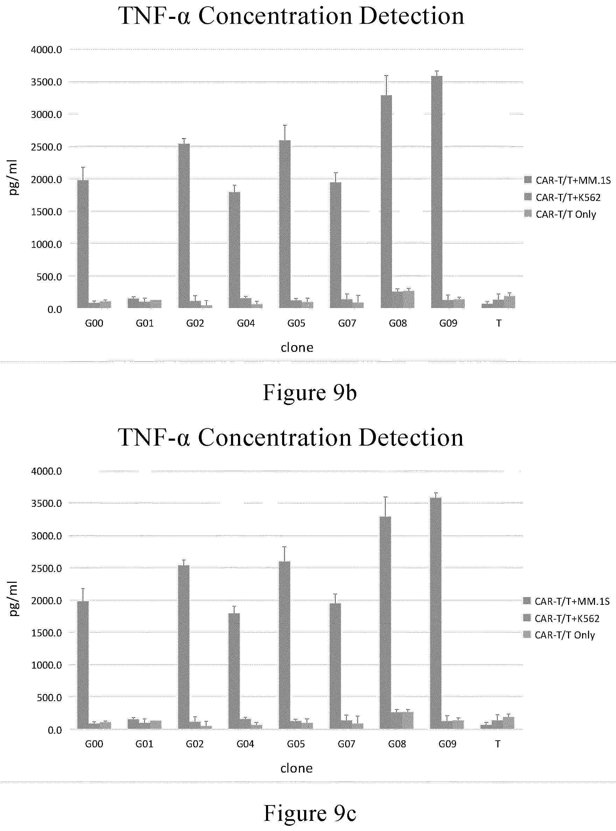

As shown in Figure 9, the concentrations of IL-2 (Figure 9a), TNF-α (Figure 9b), and IFN-γ (Figure 9c) in the supernatant were detected. It can be seen that G02, G04, G05, G07, G08, and G09 CAR-T cells specifically killed MM.1S cells and induced higher levels of inflammatory cytokines. Furthermore, all CAR-T or T cells could not kill K562 cells, indicating the specificity of CAR-T cell-mediated killing.

### Example 8: Specific Proliferation Assay of CAR-T Cells

A 24-well plate was set up with four reaction condition groups as follows:
A: CAR-T/T + NCI-H929: CAR-T cells or T cells co-cultured with multiple myeloma cell line NCI-H929.
B: CAR-T/T + K562: CAR-T cells or T cells co-cultured with K562 cells.
C: CAR-T/T only: CAR-T cells or T cells cultured alone.
D: CAR-T/T + IL-2: CAR-T cells or T cells cultured with 300 U/mL IL-2.

The G00 - G09 CAR-T cells and T cells prepared in Example 5 of the culture were washed with PBS and then resuspended in the culture medium for sampling and counting. The concentrations of CAR-T cells and T cells were adjusted to 5 - 10 × 10E6 cells/ml based on the cell concentration. CFSE staining was added to the cell suspension for 10 minutes, followed by the addition of FBS to terminate the staining. The supernatant was removed by centrifugation, and the cells were washed multiple times with the complete culture medium. The cells were resuspended in the culture medium and sampled for counting. The concentration of CAR-positive cells or T cells in the cell suspension was adjusted to 1 × 10E6 cells/ml. CAR-T cells and T cells were respectively added to the labeled 24-well plates, with 100 µl of the cell suspension per well.

NCI-H929 and K562 cells in culture were washed with PBS, resuspended in culture medium, and counted. The cell concentration was adjusted to 1*10^6 cells/mL, and the NCI-H929 cell suspension was added to the wells of group A (CAR-T/T + NCI-H929) at 200 µL per well. The K562 cell suspension was added to the wells of group B (CAR-T/T + K562) at 200 µL per well. For group C (CAR-T/T only), 200 µL of culture medium without IL-2 was added, and for group D (CAR-T/T + IL-2), 200 µL of culture medium with IL-2 was added. The wells containing the cell suspensions were mixed well and incubated overnight in a CO₂ incubator for 120 hours.

After incubation, the cell suspensions were collected, washed with PBS, and stained for flow cytometry. CD3 and CAR staining was performed. For CAR-T cells, CD3-positive cells were selected, and CAR-positive cells were identified, with the FITC peaks overlaid. For T cells, CD3-positive cells were selected, and the FITC peaks were similarly analyzed.

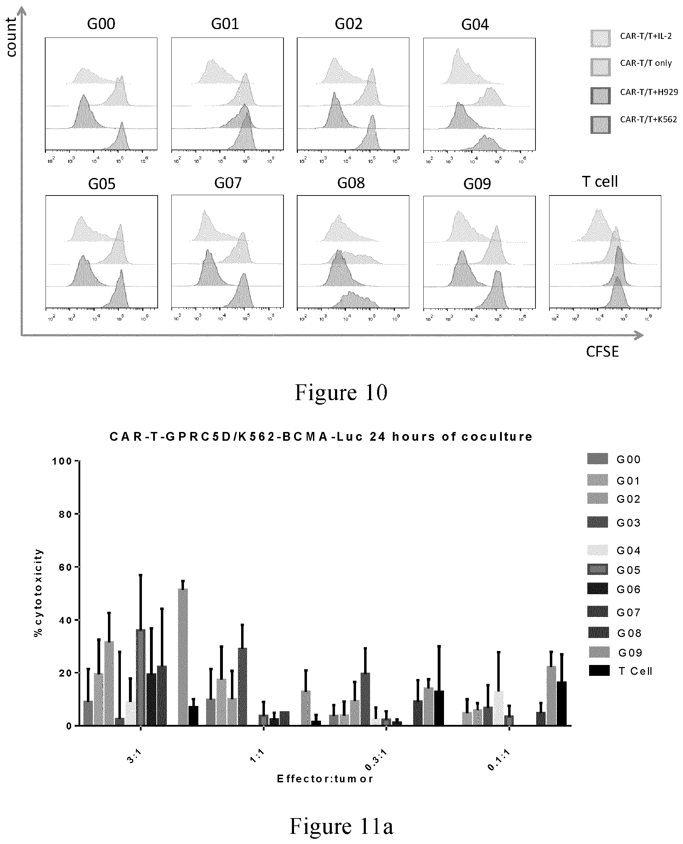

As shown in Figure 10, the CFSE fluorescence intensity reduction in each CAR-T cell group after co-culture with target cells is displayed. The results indicate that G02, G04, G05, G07, and G09 CAR-T cells specifically recognize NCI-H929 cells and undergo specific proliferation. However, G01 CAR-T and T cells did not specifically recognize NCI-H929 cells, and thus did not show specific proliferation. G08 CAR-T cells exhibited proliferation regardless of whether IL-2 was present in the culture medium or whether they were co-cultured with K562 or NCI-H929 cells. This indicates that the scFv in G08 CAR-T cells caused high levels of cell auto-activation.

### Example 9: Specific Cytotoxicity Assay of CAR-T Cells

The G00~G09 CAR-T cells and T cells were washed with PBS and resuspended in culture medium for counting. The concentration of CAR-positive cells and T cells was adjusted to 3×10^5 cells/mL, 1×10^5 cells/mL, 0.3×10^5 cells/mL, and 0.1×10^5 cells/mL. The diluted CAR-T cells or T cells were added to the corresponding wells of a 96-well plate, with 100 µL per well.

The target cells used in the experiment were: K562-luc, K562-GPRC5D-luc, NCI-H929-luc, RPMI 8226-luc, and MM.1S-luc. These cells were washed with PBS and resuspended in culture medium. The concentration of target cells was adjusted to 1x10^5 cells/mL. The target cell suspension was added to the corresponding wells of the 96-well plate, with 100 µL per well. The plate was then incubated overnight in a CO₂ incubator for 24 hours.

After incubation, luciferase substrate solution was added to each well. The plate was then placed in a multifunctional microplate reader for bioluminescence detection.

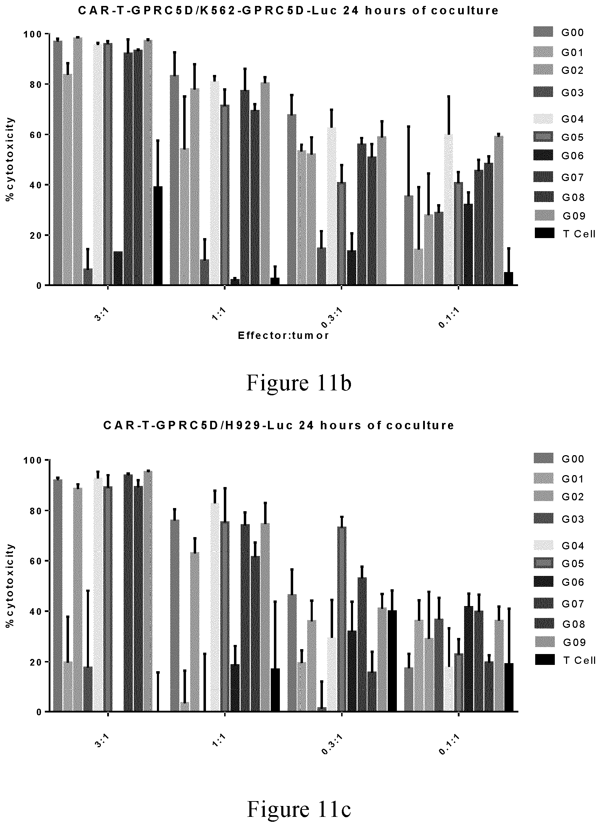

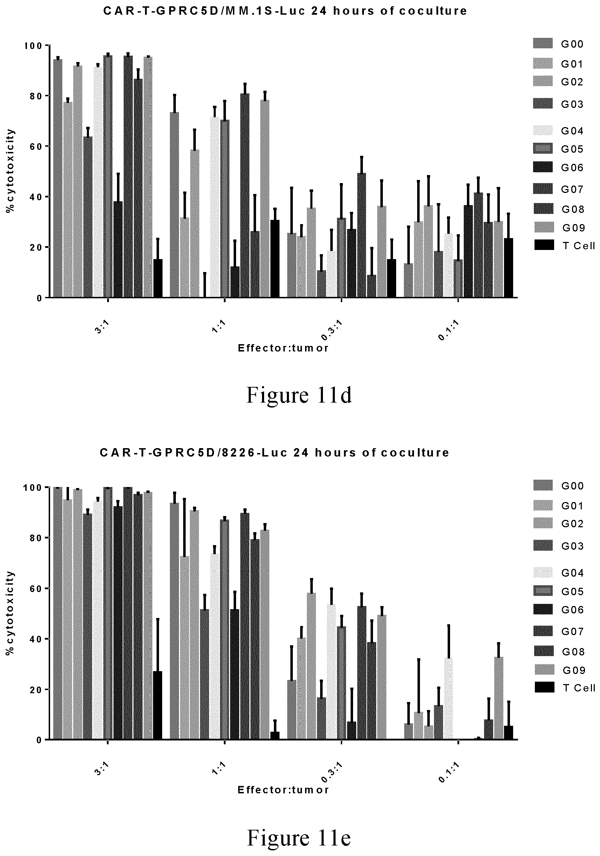

As shown in Figure 11, the results indicate that G00, G02, G05, G07, and G09 CAR-T cells can effectively kill GPRC5D-positive target cells.

### Example 10: In Vivo Efficacy Test of GPRC5D-CAR-T

This experiment involved the intravenous injection of the test substance GPRC5D-CAR-T into NOD-Prkdcscid Il2rgtm1/Bcgen mice (B-NDG mice) transplanted with human multiple myeloma cells MM.1S, to evaluate its *in vivo* inhibitory effect on the proliferation of tumor cells.

### Methods:

1) Screening and Grouping: Forty female B-NDG mice were intravenously injected with 1×10⁶ MM.1S-Luc (luciferase-labeled human MM.1S cells) for 5 days. The mice were then randomly divided into 8 groups: vehicle control group (Vehicle), negative control MOCK-T group (T mock), test groups with different doses of GPRC5D-CAR-T cells, as follows: G05 1E6 treatment group, G05 5E6 treatment group, G07 1E6 treatment group, G07 5E6 treatment group, G09 1E6 treatment group, G09 5E6 treatment group. Each group consisted of 5 mice.
2) Cell Implantation: Each mouse was intravenously injected with 1×10⁶ MM.1S-Luc cells (luciferase-labeled human MM.1S cells). The injection was followed by treatment on Day 5.
3) Administration: Mice in Vehicle control group (Vehicle) were received with a solvent solution; Mice in MOCK-T group were received with negative control MOCK-T cells; Mice in G05 1E6 group were received with 1×10⁶G05 CAR-T cells per mouse; Mice in G05 5E6 group were received with 5×10⁶ G05 CAR-T cells per mouse; Mice in G07 1E6 group were received with 1×10⁶G07 CAR-T cells per mouse; Mice in G07 5E6 group were received with 5×10⁶ G07 CAR-T cells per mouse; Mice in G09 1E6 group were received with 1×10⁶G09 CAR-T cells per mouse; Mice in G09 5E6 group were received with 5×10⁶ G09 CAR-T cells per mouse. The day of the first administration is D0, and all treatments were administered via a single intravenous injection.
4) Detection Indicators: Imaging was performed using the Bruker small animal imaging system at the following time points: Day -5 (5 days before administration), Day -1 (1 day before administration), Day 3, Day 6, Day 10, Day 13, Day 18, and Day 24. Chemiluminescence signals from all animals were captured at each time point.

### Conclusion:

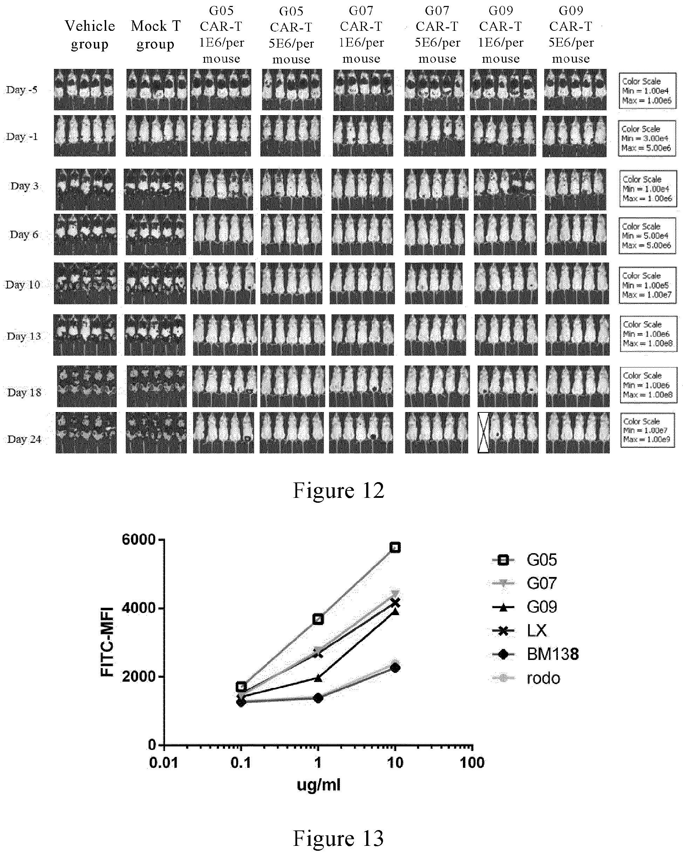

The results, shown in Figure 12, indicate that compared to the low-dose groups (G05 1E6, G07 1E6, G09 1E6), the high-dose groups (G05 5E6, G07 5E6, G09 5E6) exhibited superior therapeutic efficacy.

### Example 11: Endocytosis Effect of GPRC5D Antibody

The reference clone LX was set up, with the antibody sequence sourced from the patent (CN 115038720 A) of Shanghai Lixin Pharmaceutical Research and Development Co., Ltd. The unrelated control antibody BM138, targeting CLDN18.2, is zolbetuximab. After purifying the antibodies, their concentration was measured using the BCA assay, and the antibodies were diluted for the experiment. The antibody dilution was prepared in culture medium at concentrations of 40 µg/ml, 4 µg/ml, and 0.4 µg/ml. For each dilution, 25 µl was added to each well, with three replicates per dilution. Wells without antibody were set up as controls. The Zenon pHrodo iFL Green IgG labeling reagent was diluted with culture medium to final concentrations of 40 µg/ml, 4 µg/ml, and 0.4 µg/ml. Each dilution was added to corresponding wells containing the antibody dilution at the same concentration (25 µl per well), and mixed well. Control wells were prepared with only the labeling reagent at varying concentrations but without any antibody. The plates were incubated at room temperature for 10 minutes, then 50 µl of K562-GPRC5D cell suspension was added to each well (50000 cells per well). The plate was incubated for 24 hours, and flow cytometry was used to detect the MFI (mean fluorescence intensity) of cells in each well. The MFI values of different antibodies at different concentrations were recorded for statistical analysis.

As shown in Figure 13, the MFI curves for G05-mAb, G07-mAb, and G09-mAb indicate that these three clones demonstrated good endocytosis at all tested antibody concentrations.

### Example 12: In vitro Functional Characterization of GPRC5D&CD3 Bispecific Antibody

### 12.1 Production and Detection of GPRC5D&CD3 Bispecific Antibody Vectors

The CD3 antibody used in this example is a humanized antibody derived from the mouse hybridoma SP34. The antibody sequence is from patent CN109715667A, specifically the CD3B219 VH and CD3B219 VL regions, with codon optimization and gene synthesis performed for CD3VH and CD3VL. The CD3VH was cloned into the expression vector pTT5-hIgG1 FC-Knob, and the CD3VL was cloned into the expression vector pTT5-hKappa. The ScFv of GPRC5D antibodies (G05, G07, G09, G00) were cloned into the expression vector pTT5-hIgG1 FC-Hole. After PCR amplification, restriction enzyme digestion, ligation, transformation, and plasmid preparation, a total of 6 expression plasmids were obtained: pTT5-CD3VH-Knob, pTT5-CD3VL, pTT5-G05-Hole, pTT5-G07-Hole, pTT5-G09-Hole, and pTT5-G00-Hole.

The plasmids pTT5-CD3VH-Knob and pTT5-CD3VL were co-transfected into 293F cells with the four plasmids pTT5-G05-Hole, pTT5-G07-Hole, pTT5-G09-Hole, and pTT5-G00-Hole, respectively, for antibody expression. After collecting the cell supernatants expressing G05 BsAb, G07 BsAb, G09 BsAb, and G00 BsAb, protein purification was performed using Protein A affinity chromatography. After PBS dialysis to remove impurities, the protein concentrations were determined.

### 12.2 In vitro Functional Characterization of Bispecific Antibodies

### 12.2.1 Flow Cytometry Detection of Bispecific Antibody Binding Activity

NCI-H929 and Jurkat cells were cultured for 48 hours, counted, and plated at a density of 2 × 10⁵ cells in 96-well V-bottom plates. The bispecific antibodies were serially diluted in PBS, with Talquetamab used as a positive control. The starting concentration was 100 µg/ml, followed by 1:5, 1:2, 1:2, 1:2, 1:2, 1:2, 1:2, 1:2 serial dilutions, resulting in 8 concentrations for testing. 100 µl of proteins was added to each well with 200,000 cells. The BsAb was incubated with cells at 4°C for 1 hour, washed three times with PBS and 0.2% FBS, and then APC-conjugated anti-human IgG mAb was added to each well. The cells were incubated with the secondary antibody for 1 hour at 4°C, washed three times again with PBS and 0.2% FBS, and then analyzed using flow cytometry.

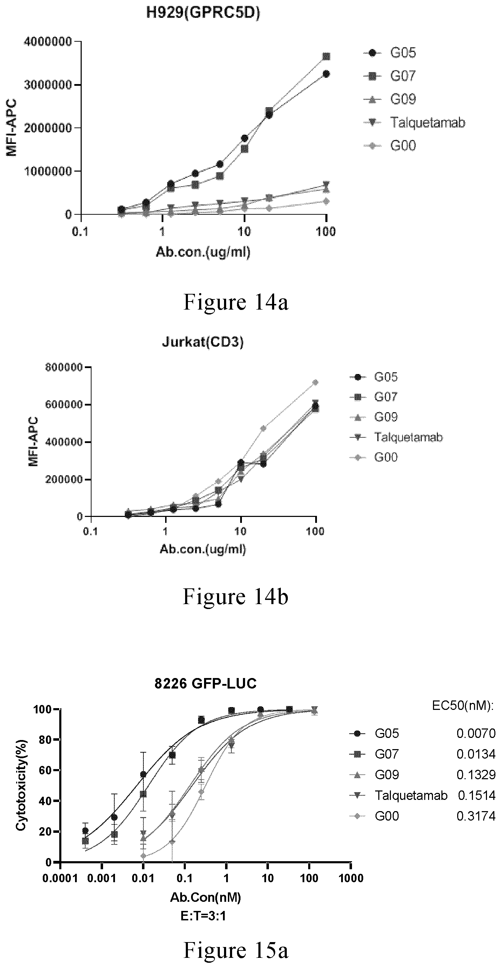

As shown in Figures 14a and 14b, all bispecific antibodies bound to NCI-H929 cells expressing endogenous GPRC5D in a dose-dependent manner. Among them, G05 and G07 demonstrated significantly higher affinity compared to G09, G00, and Talquetamab.

### 12.2.2 Bispecific Antibody-Mediated T Cell Killing of Tumor Cells

The bispecific antibody-mediated cytotoxicity of T cells was analyzed using NCI-H929-GFP-LUC, MM.1S-GFP-LUC, and RPMI-8226-GFP-LUC cells. Target cells (NCI-H929-GFP-LUC, MM.1S-GFP-LUC, RPMI-8226-GFP-LUC) were washed twice in X-Vivo killing medium containing 5% FBS. The cells were diluted to a concentration of 2 × 10⁵/mL in X-Vivo killing medium and incubated at 37°C until use.

Normal donor PBMCs were thawed and CD3-positive T cells were isolated using CD3 magnetic beads. Based on cell counts, T cells were resuspended in complete T cell culture medium (X-Vivo + 5% FBS + 200 IU/ml IL-2 + 1% PS) at a concentration of 1.5-2.5 × 10⁶/ml. After 1 week of expansion, the supernatant was discarded and the T cells were resuspended at 1.0 × 10⁶/mL in X-Vivo killing medium.

2 × 10⁴ target cells were added to each well of a 96-well plate, followed by the addition of 6 × 10⁴ T cells (3:1 effector-to-target ratio). After mixing, 20 µl of the GPRC5D×CD3 bispecific antibody dilution was added to each well. The bispecific antibody was initially diluted to a concentration of 20 µg/ml in PBS and added to the wells containing target and T cells. A 5-fold series of dilutions was prepared in PBS for antibody titration, and the final column was left for PBS-only (vector control). The plates were incubated at 37°C and 5% CO₂ for 48 hours.

After two days (48 hours), the plates were centrifuged, and 100 µl of the supernatant was stored at -80°C for cytokine release assays. 100 µl of a 30 mg/ml luciferin solution was added and incubated at 37°C for 5 minutes, followed by bioluminescence detection using a microplate reader. The cell killing effect of the bispecific antibodies on target cells was analyzed based on luciferase activity. Data were plotted and fitted using nonlinear regression with a variable slope (four-parameter) function in GraphPad Prism 8.

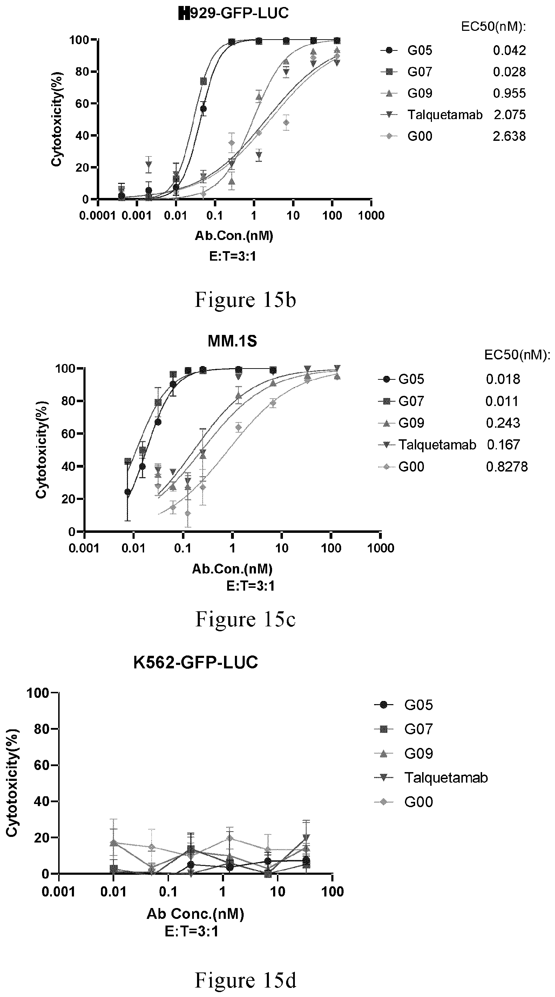

As shown in Figures 15a-d, all GPRC5D&CD3 bispecific antibodies exhibited dose-dependent killing of multiple myeloma cell lines (NCI-H929, MM.1S, RPMI-8226) that endogenously express GPRC5D. G05 and G07 showed significantly higher killing efficiency compared to G00, G09, and Talquetamab. None of the bispecific antibodies showed significant killing activity against the negative control K562 cell line.

### The sequences of the present invention.

| No. | Sequence Name | sequence | SEQ ID NO: |
|---|---|---|---|
| G01 | VH-CDR1 | LSSNAMS | 1 |
| | VH-CDR2 | SVGSSGRPYYAN | 2 |
| | VH-CDR3 | FTGGTSGGYFSI | 3 |
| | VL-CDR1 | QASQSVYNNNLLA | 4 |
| | VL-CDR2 | SAST | 5 |
| | VL-CDR3 | QGYYSGAICT | 6 |
| | VH Amino acid | EVQLVESGGGLVQPGGSLRLSCTASGFSLSSNAMSWVRQAPGKGLEW IGSVGSSGRPYYANWAKGRFTISRDNSKNTVYLQMNSLRAEDTAVYF CARFTGGTSGGYFSIWGPGTLVTVSS | 7 |
| | VH Nucleic acid | GAAGTGCAGCTGGTGGAGTCCGGCGGTGGACTGGTGCAACCGGGA GGCTCACTCAGATTGTCATGCACCGCCTCTGGCTTTAGTCTCAGTAG TAATGCAATGAGCTGGGTGAGGCAGGCACCCGGCAAGGGCCTGGA ATGGATCGGCAGCGTTGGTAGTAGTGGTAGACCATACTACGCGAAC TGGGCGAAGGGCAGGTTTACCATCAGCCGCGACAACAGCAAGAAT ACCGTTTACCTGCAGATGAATAGCCTGAGGGCCGAAGACACGGCG GTCTATTTCTGTGCACGGTTTACTGGTGGTACTAGTGGGGGTTACTT TTCCATCTGGGGCCCTGGGACCCTCGTAACTGTGAGCAGC | 8 |
| | VL Amino acid | DIQMTQSPSTLSASVGDRVIINCQASQSVYNNNLLAWYQQKPGKAPK LLIYSASTLASGVPSRFSGSGSGAEFTLTISSLQPDDFATYYCQGYYSGA ICTFGQGTKLTVL | 9 |
| | VL Nucleic acid | GACATCCAGATGACCCAGTCCCCAAGTACACTGAGCGCCTCCGTGG GCGACCGCGTGATCATAAACTGTCAGGCCAGTCAGAGTGTTTATAAT AACAACCTCTTAGCCTGGTATCAACAGAAGCCCGGTAAGGCCCCCA AACTGCTCATTTACTCTGCATCCACTCTGGCATCCGGGGTGCCAAGC CGCTTCTCCGGGAGTGGGTCTGGCGCCGAGTTCACCCTGACCATAT CTTCCCTGCAGCCCGACGACTTCGCAACGTACTATTGCCAAGGCTA | 10 |
| | | CTATAGTGGTGCTATTTGTACTTTCGGGCAGGGCACGAAGTTGACCG TGCTG | |
| | scFv | DIQMTQSPSTLSASVGDRVIINCQASQSVYNNNLLAWYQQKPGKAPK LLIYSASTLASGVPSRFSGSGSGAEFTLTISSLQPDDFATYYCQGYYSGA ICTFGQGTKLTVLGGGGSGGGGSGGGGSEVQLVESGGGLVQPGGSLR LSCTASGFSLSSNAMSWVRQAPGKGLEWIGSVGSSGRPYYANWAKGR FTISRDNSKNTVYLQMNSLRAEDTAVYFCARFTGGTSGGYFSIWGPGT LVTVSS | 11 |
| G02 | VH-CDR1 | LSSNAMS | 1 |
| | VH-CDR2 | SIGRSGSTYNAN | 12 |
| | VH-CDR3 | KFTGGVSGGYFSV | 13 |
| | VL-CDR1 | QASQSVYNNNLLS | 14 |
| | VL-CDR2 | AASN | 15 |
| | VL-CDR3 | QGYYGGAICA | 16 |
| | VH Amino acid | EVQLVESGGGLVQPGGSLRLSCTASGFSLSSNAMSWVRQAPGKGLEW IGSIGRSGSTYNANWAKGRFTISRDNSKNTVYLQMNSLRAEDTAVYFC AKFTGGVSGGYFSVWGPGTLVTVSS | 17 |
| | VH Nucleic acid | GAAGTGCAGCTGGTGGAGTCCGGCGGTGGACTGGTGCAACCGGGA GGCTCACTCAGATTGTCATGCACCGCCTCTGGCTTTAGTCTCAGTAG TAATGCAATGAGCTGGGTGAGGCAGGCACCCGGCAAGGGCCTGGA ATGGATCGGCAGCATTGGTCGTAGTGGTAGCACATACAACGCGAAC TGGGCGAAGGGCAGGTTTACCATCAGCCGCGACAACAGCAAGAAT ACCGTTTACCTGCAGATGAATAGCCTGAGGGCCGAAGACACGGCG GTCTATTTCTGTGCAAAATTTACTGGTGGTGTTAGTGGGGGTTACTT TAGCGTCTGGGGCCCTGGGACCCTCGTAACTGTGAGCAGC | 18 |
| | VL Amino acid | DIQMTQSPSTLSASVGDRVIINCQASQSVYNNNLLSWYQQKPGKAPKL LIYAASNLASGVPSRFSGSGSGAEFTLTISSLQPDDFATYYCQGYYGGA ICAFGQGTKLTVL | 19 |
| | VL Nucleic acid | GACATCCAGATGACCCAGTCCCCAAGTACACTGAGCGCCTCCGTGG GCGACCGCGTGATCATAAACTGTCAGGCCAGTCAGAGTGTTTATAAT AACAACCTCTTATCTTGGTATCAACAGAAGCCCGGTAAGGCCCCCA AACTGCTCATTTACGCTGCATCCAATCTGGCATCCGGGGTGCCAAGC CGCTTCTCCGGGAGTGGGTCTGGCGCCGAGTTCACCCTGACCATAT CTTCCCTGCAGCCCGACGACTTCGCAACGTACTATTGCCAAGGCTA CTATGGTGGTGCTATTTGTGCTTTCGGGCAGGGCACGAAGTTGACC GTGCTG | 20 |
| | scFv | DIQMTQSPSTLSASVGDRVIINCQASQSVYNNNLLSWYQQKPGKAPKL LIYAASNLASGVPSRFSGSGSGAEFTLTISSLQPDDFATYYCQGYYGGA ICAFGQGTKLTVLGGGGSGGGGSGGGGSEVQLVESGGGLVQPGGSLR LSCTASGFSLSSNAMSWVRQAPGKGLEWIGSIGRSGSTYNANWAKGR FTISRDNSKNTVYLQMNSLRAEDTAVYFCAKFTGGVSGGYFSVWGPG TLVTVSS | 21 |
| G03 | VH-CDR1 | IDFSSSYWIC | 22 |
| | VH-CDR2 | ACIANGDGSTYYAS | 23 |
| | VH-CDR3 | DPYLGYSVQLNL | 24 |
| | VL-CDR1 | QASEDIGNNLA | 25 |
| | VL-CDR2 | KAST | 26 |
| | VL-CDR3 | QCTTYYGSGYGVGA | 27 |
| | VH Amino acid | EVQLVESGGGLVQPGGSLRLSCTASGIDFSSSYWICWVRQAPGKGLE WIACIANGDGSTYYASWAKGRFTISRDNSKNTVYLQMNSLRAEDTAV YFCARDPYLGYSVQLNLWGPGTLVTVSS | 28 |
| | VH Nucleic acid | GAAGTGCAGCTGGTGGAGTCCGGCGGTGGACTGGTGCAACCGGGA GGCTCACTCAGATTGTCATGCACCGCCTCTGGCATCGACTTCAGTAG CAGCTACTGGATATGCTGGGTGAGGCAGGCACCCGGCAAGGGCCT GGAATGGATCGCATGCATTGCTAATGGTGATGGCAGCACATACTACG CGAGCTGGGCGAAGGGCAGGTTTACCATCAGCCGCGACAACAGCA | 29 |
| | | AGAATACCGTTTACCTGCAGATGAATAGCCTGAGGGCCGAAGACAC GGCGGTCTATTTCTGTGCACGGGATCCTTATCTTGGTTATAGTGTTCA ACTTAACTTGTGGGGCCCTGGGACCCTCGTAACTGTGAGCAGC | |
| | VL Amino acid | DIQMTQSPSTLSASVGDRVIINCQASEDIGNNLAWYQQKPGKAPKLLI YKASTLASGVPSRFSGSGSGAEFTLTISSLQPDDFATYYCQCTTYYGSG YGVGAFGQGTKLTVL | 30 |
| | VL Nucleic acid | GACATCCAGATGACCCAGTCCCCAAGTACACTGAGCGCCTCCGTGG GCGACCGCGTGATCATAAACTGTCAGGCCAGTGAGGATATTGGTAA TAATTTAGCCTGGTATCAACAGAAGCCCGGTAAGGCCCCCAAACTG CTCATTTACAAGGCATCCACTCTGGCATCCGGGGTGCCAAGCCGCT TCTCCGGGAGTGGGTCTGGCGCCGAGTTCACCCTGACCATATCTTC CCTGCAGCCCGACGACTTCGCAACGTACTATTGCCAATGTACTACTT ATTATGGTAGTGGTTATGGGGTTGGGGCTTTCGGGCAGGGCACGAA GTTGACCGTGCTG | 31 |
| | scFv | DIQMTQSPSTLSASVGDRVIINCQASEDIGNNLAWYQQKPGKAPKLLI YKASTLASGVPSRFSGSGSGAEFTLTISSLQPDDFATYYCQCTTYYGSG YGVGAFGQGTKLTVLGGGGSGGGGSGGGGSEVQLVESGGGLVQPGG SLRLSCTASGIDFSSSYWICWVRQAPGKGLEWIACIANGDGSTYYASW AKGRFTISRDNSKNTVYLQMNSLRAEDTAVYFCARDPYLGYSVQLNL WGPGTLVTVSS | 32 |
| G04 | VH-CDR1 | LSSNAIS | 33 |
| | VH-CDR2 | IITTGGNAYYAG | 34 |
| | VH-CDR3 | GYGIHNF | 35 |
| | VL-CDR1 | KCQASESISNYLS | 36 |
| | VL-CDR2 | RAST | 37 |
| | VL-CDR3 | QGYDGSSSSSNYESA | 38 |
| | VH Amino acid | EVQLVESGGGLVQPGGSLRLSCAASGFSLSSNAISWVRQAPGKGLEWI GIITTGGNAYYAGWAKGRFTISRDNSKNTLYLQMNSLRAEDTAVYYC ARGYGIHNFWGPGTLVTVSS | 39 |
| | VH Nucleic acid | GAAGTGCAGCTGGTGGAGTCCGGCGGTGGACTGGTGCAACCGGGA GGCTCACTCAGATTGTCATGCGCCGCCTCTGGCTTTAGTCTCAGTAG CAATGCAATCAGCTGGGTGAGGCAGGCACCCGGCAAGGGCCTGGA ATGGATCGGCATCATTACTACTGGTGGTAATGCATACTACGCAGGCT GGGCGAAGGGCAGGTTTACCATCAGCCGCGACAACAGCAAGAATA CCCTGTACCTGCAGATGAATAGCCTGAGGGCCGAAGACACGGCGGT CTATTATTGTGCACGGGGGTATGGTATTCATAACTTCTGGGGCCCTG GGACCCTCGTAACTGTGAGCAGC | 40 |
| | VL Amino acid | DIQMTQSPSSLSASVGDRVTIKCQASESISNYLSWYQQKPGKVPKLLIY RASTLASGVPSRFSGSGSGTDFTLTISSLQPEDVATYYCQGYDGSSSSS NYESAFGQGTKLTVL | 41 |
| | VL Nucleic acid | GACATCCAGATGACCCAGTCCCCAAGTTCCCTGAGCGCCTCCGTGG GCGACCGCGTGACCATAAAGTGCCAGGCCAGTGAAAGCATTAGCA ACTACTTATCCTGGTATCAACAGAAGCCCGGTAAGGTGCCCAAACT GCTCATTTACAGGGCATCCACTCTGGCATCCGGGGTGCCAAGCCGC TTCTCCGGGAGTGGGTCTGGCACCGACTTCACCCTGACCATATCTTC CCTGCAGCCCGAGGACGTGGCAACGTACTATTGCCAGGGCTATGAT GGTAGTAGTAGTAGTAGTAATTATGAGAGTGCTTTCGGGCAGGGCA CGAAGTTGACCGTGCTG | 42 |
| | scFv | DIQMTQSPSSLSASVGDRVTIKCQASESISNYLSWYQQKPGKVPKLLIY RASTLASGVPSRFSGSGSGTDFTLTISSLQPEDVATYYCQGYDGSSSSS NYESAFGQGTKLTVLGGGGSGGGGSGGGGSEVQLVESGGGLVQPGGS LRLSCAASGFSLSSNAISWVRQAPGKGLEWIGIITTGGNAYYAGWAKG RFTISRDNSKNTLYLQMNSLRAEDTAVYYCARGYGIHNFWGPGTLVT VSS | 43 |
| G05 | VH-CDR1 | LTDNGIT | 44 |
| | VH-CDR2 | IMTTGGSTYYAG | 45 |
| | VH-CDR3 | TYFCTRGYGIHG | 46 |
| | VL-CDR1 | QASESISKFLS | 47 |
| | VL-CDR2 | RAST | 37 |
| | VL-CDR3 | QGYDGSSTTTNYESA | 48 |
| | VH Amino acid | EVQLVESGGGLVQPGGSLRLSCAASGFSLTDNGITWVRQAPGKGLEWI GIMTTGGSTYYAGWAKGRFTISRDNSKNTLYLQMNSLRAEDTATYFC TRGYGIHGLWGPGTLVTVSS | 49 |
| | VH Nucleic acid | GAAGTGCAGCTGGTGGAGTCCGGCGGTGGACTGGTGCAACCGGGA GGCTCACTCAGATTGTCATGCGCCGCCTCTGGCTTTAGTCTCACTGA CAATGGAATAACCTGGGTGAGGCAGGCACCCGGCAAGGGCCTGGA ATGGATCGGCATTATGACTACTGGTGGTAGCACATACTACGCAGGCT GGGCGAAGGGCAGGTTTACCATCAGCCGCGACAACAGCAAGAATA CCCTGTACCTGCAGATGAATAGCCTGAGGGCCGAAGACACGGCGA CCTATTTCTGTACCAGAGGGTATGGCATTCATGGTCTGTGGGGCCCT GGGACCCTCGTAACTGTGAGCAGCA | 50 |
| | VL Amino acid | DIQMTQSPSSLSASVGDRVTITCQASESISKFLSWYQQKPGKVPKLLIY RASTLASGVPSRFSGSGSGTDFTLTISSLQPEDVATYYCQGYDGSSTTT NYESAFGQGTKLTVL | 51 |
| | VL Nucleic acid | GACATCCAGATGACCCAGTCCCCAAGTTCCCTGAGCGCCTCCGTGG GCGACCGCGTGACCATAACCTGTCAGGCCAGTGAGAGTATTAGTAA GTTCTTATCCTGGTATCAACAGAAGCCCGGTAAGGTGCCCAAACTG CTCATTTACAGGGCATCCACTCTGGCATCCGGGGTGCCAAGCCGCT TCTCCGGGAGTGGGTCTGGCACCGACTTCACCCTGACCATATCTTCC CTGCAGCCCGAGGACGTGGCAACGTACTATTGCCAAGGTTATGATG GTAGTAGTACTACTACTAATTATGAGAGTGCTTTCGGGCAGGGCACG AAGTTGACCGTGCTG | 52 |
| | scFv | DIQMTQSPSSLSASVGDRVTITCQASESISKFLSWYQQKPGKVPKLLIY RASTLASGVPSRFSGSGSGTDFTLTISSLQPEDVATYYCQGYDGSSTTT NYESAFGQGTKLTVLGGGGSGGGGSGGGGSEVQLVESGGGLVQPGGS LRLSCAASGFSLTDNGITWVRQAPGKGLEWIGIMTTGGSTYYAGWAK GRFTISRDNSKNTLYLQMNSLRAEDTATYFCTRGYGIHGLWGPGTLVT VSS | 53 |
| G06 | VH-CDR1 | LTDNGIT | 44 |
| | VH-CDR2 | IMTTGGSAYYAG | 54 |
| | VH-CDR3 | TRGYGIH | 55 |
| | VL-CDR1 | QASESISKFLS | 47 |
| | VL-CDR2 | RAST | 37 |
| | VL-CDR3 | QGYDGSSTTTNYESA | 48 |
| | VH Amino acid | EVQLVESGGGLVQPGGSLRLSCAASGFSLTDNGITWVRQAPGKGLEWI GIMTTGGSAYYAGWAKGRFTISRDNSKNTLYLQMNSLRAEDTAVYYC ARTRGYGIHLWGPGTLVTVSS | 56 |
| | VH Nucleic acid | GAAGTGCAGCTGGTGGAGTCCGGCGGTGGACTGGTGCAACCGGGA GGCTCACTCAGATTGTCATGCGCCGCCTCTGGCTTTAGTCTCACTGA CAATGGAATAACCTGGGTGAGGCAGGCACCCGGCAAGGGCCTGGA ATGGATCGGCATTATGACTACTGGTGGTAGCGCATACTACGCAGGCT GGGCGAAGGGCAGGTTTACCATCAGCCGCGACAACAGCAAGAATA CCCTGTACCTGCAGATGAATAGCCTGAGGGCCGAAGACACGGCGGT CTATTATTGTGCACGGACCAGAGGGTATGGCATTCATCTGTGGGGCC CTGGGACCCTCGTAACTGTGAGCAGC | 57 |
| | VL Amino acid | DIQMTQSPSSLSASVGDRVTITCQASESISKFLSWYQQKPGKVPKLLIY RASTLASGVPSRFSGSGSGTDFTLTISSLQPEDVATYYCQGYDGSSTTT NYESAFGQGTKLTVL | 51 |
| | VL Nucleic acid | GACATCCAGATGACCCAGTCCCCAAGTTCCCTGAGCGCCTCCGTGG GCGACCGCGTGACCATAACCTGTCAGGCCAGTGAAAGCATTAGTAA GTTCTTATCCTGGTATCAACAGAAGCCCGGTAAGGTGCCCAAACTG CTCATTTACAGGGCATCCACTCTGGCATCCGGGGTGCCAAGCCGCT TCTCCGGGAGTGGGTCTGGCACCGACTTCACCCTGACCATATCTTCC CTGCAGCCCGAGGACGTGGCAACGTACTATTGCCAAGGTTATGATG | 52 |
| | | GTAGTAGTACTACTACAAATTATGAGAGTGCTTTCGGGCAGGGCAC GAAGTTGACCGTGCTG | |
| | scFv | DIQMTQSPSSLSASVGDRVTITCQASESISKFLSWYQQKPGKVPKLLIY RASTLASGVPSRFSGSGSGTDFTLTISSLQPEDVATYYCQGYDGSSTTT NYESAFGQGTKLTVLGGGGSGGGGSGGGGSEVQLVESGGGLVQPGGS LRLSCAASGFSLTDNGITWVRQAPGKGLEWIGIMTTGGSAYYAGWAK GRFTISRDNSKNTLYLQMNSLRAEDTAVYYCARTRGYGIHLWGPGTLV TVSS | 58 |
| G07 | VH-CDR1 | LTDNGIT | 44 |
| | VH-CDR2 | IMTTGGTTYYAG | 59 |
| | VH-CDR3 | GYGIHG | 60 |
| | VL-CDR1 | QASESVSKFLS | 61 |
| | VL-CDR2 | RAST | 37 |
| | VL-CDR3 | QGYDGSSTTTNYESA | 48 |
| | VH Amino acid | EVQLVESGGGLVQPGGSLRLSCAASGFSLTDNGITWVRQAPGKGLEWI GIMTTGGTTYYAGWAKGRFTISRDNSKNTLYLQMNSLRAEDTAVYYC ARGYGIHGLWGPGTLVTVSS | 62 |
| | VH Nucleic acid | GAAGTGCAGCTGGTGGAGTCCGGCGGTGGACTGGTGCAACCGGGA GGCTCACTCAGATTGTCATGCGCCGCCTCTGGCTTTAGTCTCACTGA CAATGGAATAACCTGGGTGAGGCAGGCACCCGGCAAGGGCCTGGA ATGGATCGGCATTATGACTACTGGTGGTACCACATACTACGCAGGCT GGGCGAAGGGCAGGTTTACCATCAGCCGCGACAACAGCAAGAATA CCCTGTACCTGCAGATGAATAGCCTGAGGGCCGAAGACACGGCGGT CTATTATTGTGCACGGGGGTATGGCATTCATGGTCTGTGGGGCCCTG GGACCCTCGTAACTGTGAGCAGC | 63 |
| | VL Amino acid | DIQMTQSPSSLSASVGDRVTITCQASESVSKFLSWYQQKPGKVPKLLIY RASTLASGVPSRFSGSGSGTDFTLTISSLQPEDVATYYCQGYDGSSTTT NYESAFGQGTKLTVL | 64 |
| | VL Nucleic acid | GACATCCAGATGACCCAGTCCCCAAGTTCCCTGAGCGCCTCCGTGG GCGACCGCGTGACCATAACCTGTCAGGCCAGTGAAAGCGTTAGTAA GTTCTTATCCTGGTATCAACAGAAGCCCGGTAAGGTGCCCAAACTG CTCATTTACAGGGCATCCACTCTGGCATCCGGGGTGCCAAGCCGCT TCTCCGGGAGTGGGTCTGGCACCGACTTCACCCTGACCATATCTTCC CTGCAGCCCGAGGACGTGGCAACGTACTATTGCCAAGGTTATGATG GTAGTAGTACTACTACTAATTATGAGAGTGCTTTCGGGCAGGGCACG AAGTTGACCGTGCTG | 65 |
| | scFv | DIQMTQSPSSLSASVGDRVTITCQASESVSKFLSWYQQKPGKVPKLLIY RASTLASGVPSRFSGSGSGTDFTLTISSLQPEDVATYYCQGYDGSSTTT NYESAFGQGTKLTVLGGGGSGGGGSGGGGSEVQLVESGGGLVQPGGS LRLSCAASGFSLTDNGITWVRQAPGKGLEWIGIMTTGGTTYYAGWAK GRFTISRDNSKNTLYLQMNSLRAEDTAVYYCARGYGIHGLWGPGTLV TVSS | 66 |
| G08 | VH-CDR1 | LSTYAVS | 67 |
| | VH-CDR2 | IISRSGNTYYAS | 68 |
| | VH-CDR3 | PRIFNSGDIAYLSRLD | 69 |
| | VL-CDR1 | QASQSVYSNALS | 70 |
| | VL-CDR2 | DAST | 71 |
| | VL-CDR3 | AGGYNSIVDNP | 72 |
| | VH Amino acid | EVQLVESGGGLVQPGGSLRLSCAASGFSLSTYAVSWVRQAPGKGLEWI GIISRSGNTYYASWAKGRFTISRDNSKNTLYLQMNSLRAEDTAVYYCA RPRIFNSGDIAYLSRLDLWGPGTLVTVSS | 73 |
| | VH Nucleic acid | GAAGTGCAGCTGGTGGAGTCCGGCGGTGGACTGGTGCAACCGGGA GGCTCACTCAGATTGTCATGCGCCGCCTCTGGCTTTAGTCTCAGTAC CTATGCAGTGAGCTGGGTGAGGCAGGCACCCGGCAAGGGCCTGGA ATGGATCGGCATCATTAGTCGTAGTGGTAACACATACTACGCGAGCT GGGCGAAGGGCAGGTTTACCATCAGCCGCGACAACAGCAAGAATA | 74 |
| | | CCCTGTACCTGCAGATGAATAGCCTGAGGGCCGAAGACACGGCGGT CTATTATTGTGCACGGCCAAGGATATTTAATAGTGGTGATATTGCCTA TCTAAGTCGGTTGGATCTGTGGGGCCCTGGGACCCTCGTAACTGTG AGCAGC | |
| | VL Amino acid | DIQMTQSPSSLSASVGDRVTITCQASQSVYSNALSWYQQKPGKVPKLL IYDASTLASGVPSRFSGSGSGTDFTLTISSLQPEDVATYYCAGGYNSIVD NPFGQGTKLTVL | 75 |
| | VL Nucleic acid | GACATCCAGATGACCCAGTCCCCAAGTTCCCTGAGCGCCTCCGTGG GCGACCGCGTGACCATAACCTGTCAGGCCAGTCAGAGTGTTTATAG TAACGCCTTATCCTGGTATCAACAGAAGCCCGGTAAGGTGCCCAAA CTGCTCATTTACGATGCATCCACTCTGGCATCCGGGGTGCCAAGCCG CTTCTCCGGGAGTGGGTCTGGCACCGACTTCACCCTGACCATATCTT CCCTGCAGCCCGAGGACGTGGCAACGTACTATTGCGCAGGCGGTTA CAATAGTATTGTTGATAATCCTTTCGGGCAGGGCACGAAGTTGACCG TGCTG | 76 |
| | scFv | DIQMTQSPSSLSASVGDRVTITCQASQSVYSNALSWYQQKPGKVPKLL IYDASTLASGVPSRFSGSGSGTDFTLTISSLQPEDVATYYCAGGYNSIVD NPFGQGTKLTVLGGGGSGGGGSGGGGSEVQLVESGGGLVQPGGSLRL SCAASGFSLSTYAVSWVRQAPGKGLEWIGIISRSGNTYYASWAKGRFTI SRDNSKNTLYLQMNSLRAEDTAVYYCARPRIFNSGDIAYLSRLDLWGP GTLVTVSS | 77 |
| G09 | VH-CDR1 | LSNNGIT | 78 |
| | VH-CDR2 | IITTTGSAYYAG | 79 |
| | VH-CDR3 | GYGIHG | 60 |
| | VL-CDR1 | QASESISNYLS | 80 |
| | VL-CDR2 | RAST | 37 |
| | VL-CDR3 | QGYDGSASSSNYESA | 81 |
| | VH Amino acid | EVQLVESGGGLVQPGGSLRLSCAASGFSLSNNGITWVRQAPGKGLEW IGIITTGSAYYAGWAKGRFTISRDNSKNTLYLQMNSLRAEDTAVYYC ARGYGIHGLWGPGTLVTVSS | 82 |
| | VH Nucleic acid | GAAGTGCAGCTGGTGGAGTCCGGCGGTGGACTGGTGCAACCGGGA GGCTCACTCAGATTGTCATGCGCCGCCTCTGGCTTTAGTCTCAGTAA CAATGGAATAACCTGGGTGAGGCAGGCACCCGGCAAGGGCCTGGA ATGGATCGGCATTATTACTACTACTGGTAGCGCATACTACGCAGGCT GGGCGAAGGGCAGGTTTACCATCAGCCGCGACAACAGCAAGAATA CCCTGTACCTGCAGATGAATAGCCTGAGGGCCGAAGACACGGCGGT CTATTATTGTGCACGGGGGTATGGTATTCATGGTCTGTGGGGCCCTG GGACCCTCGTAACTGTGAGCAGC | 83 |
| | VL Amino acid | DIQMTQSPSSLSASVGDRVTITCQASESISNYLSWYQQKPGKVPKLLIY RASTLASGVPSRFSGSGSGTDFTLTISSLQPEDVATYYCQGYDGSASSS NYESAFGQGTKLTVL | 84 |
| | VL Nucleic acid | GACATCCAGATGACCCAGTCCCCAAGTTCCCTGAGCGCCTCCGTGG GCGACCGCGTGACCATAACCTGTCAGGCCAGTGAAAGCATTAGCAA CTACTTATCCTGGTATCAACAGAAGCCCGGTAAGGTGCCCAAACTG CTCATTTACAGGGCATCCACTCTGGCATCCGGGGTGCCAAGCCGCT TCTCCGGGAGTGGGTCTGGCACCGACTTCACCCTGACCATATCTTCC CTGCAGCCCGAGGACGTGGCAACGTACTATTGCCAAGGTTATGATG GTAGTGCTAGTAGTAGTAATTATGAGAGTGCTTTCGGGCAGGGCAC GAAGTTGACCGTGCTG | 85 |
| | scFv | DIQMTQSPSSLSASVGDRVTITCQASESISNYLSWYQQKPGKVPKLLIY RASTLASGVPSRFSGSGSGTDFTLTISSLQPEDVATYYCQGYDGSASSS NYESAFGQGTKLTVLGGGGSGGGGSGGGGSEVQLVESGGGLVQPGGS LRLSCAASGFSLSNNGITWVRQAPGKGLEWIGIITTTGSAYYAGWAKG RFTISRDNSKNTLYLQMNSLRAEDTAVYYCARGYGIHGLWGPGTLVT VSS | 86 |
| G10 | VH-CDR1 | DINSYNMQ | 87 |
| | VH-CDR2 | IIVTGGSRWYAS | 88 |
| | VH-CDR3 | GSAL | 89 |
| | VL-CDR1 | TITCQASESIYSALA | 90 |
| | VL-CDR2 | KAST | 26 |
| | VL-CDR3 | QSTYYSSSSSYLNT | 91 |
| | VH Amino acid | EVQLVESGGGLVQPGGSLRLSCAASGFDINSYNMQWVRQAPGKGLE WIGIIVTGGSRWYASWAKGRFTISRDNSKNTLYLQMNSLRAEDTAVYY CARGSALWGPGTLVTVSS | 92 |
| | VH Nucleic acid | GAAGTGCAGCTGGTGGAGTCCGGCGGTGGACTGGTGCAACCGGGA GGCTCACTCAGATTGTCATGCGCCGCCTCTGGCTTTGACATCAATAG CTACAACATGCAATGGGTGAGGCAGGCACCCGGCAAGGGCCTGGA ATGGATCGGCATCATTGTTACTGGTGGTAGTAGATGGTACGCGAGCT GGGCGAAGGGCAGGTTTACCATCAGCCGCGACAACAGCAAGAATA CCCTGTACCTGCAGATGAATAGCCTGAGGGCCGAAGACACGGCGGT CTATTATTGTGCACGGGGATCTGCCTTGTGGGGCCCTGGGACCCTCG TAACTGTGAGCAGC | 93 |
| | VLAmino acid | DIQMTQSPSSLSASVGDRVTITCQASESIYSALAWYQQKPGKVPKLLIY KASTLASGVPSRFSGSGSGTDFTLTISSLQPEDVATYYCQSTYYSSSSSY LNTFGQGTKLTVL | 94 |
| | VL Nucleic acid | GACATCCAGATGACCCAGTCCCCAAGTTCCCTGAGCGCCTCCGTGG GCGACCGCGTGACCATAACCTGTCAGGCCAGTGAGAGCATTTACAG TGCTTTGGCCTGGTATCAACAGAAGCCCGGTAAGGTGCCCAAACTG CTCATTTACAAGGCATCCACTCTGGCATCCGGGGTGCCAAGCCGCT TCTCCGGGAGTGGGTCTGGCACCGACTTCACCCTGACCATATCTTCC CTGCAGCCCGAGGACGTGGCAACGTACTATTGCCAAAGTACTTATT ATAGTAGTAGTAGTAGTTATCTTAATACTTTCGGGCAGGGCACGAAG TTGACCGTGCTG | 95 |
| | scFv | DIQMTQSPSSLSASVGDRVTITCQASESIYSALAWYQQKPGKVPKLLIY KASTLASGVPSRFSGSGSGTDFTLTISSLQPEDVATYYCQSTYYSSSSSY LNTFGQGTKLTVLGGGGSGGGGSGGGGSEVQLVESGGGLVQPGGSLR LSCAASGFDINSYNMQWVRQAPGKGLEWIGIIVTGGSRWYASWAKGR FTISRDNSKNTLYLQMNSLRAEDTAVYYCARGSALWGPGTLVTVSS | 96 |
| CD3 Anti body | VH Amino acid | EVQLLESGGGLVQPGGSLRLSCAASGFTFSTYAMNWVRQAPGKGLE WVSRIRSKYNNYATYYADSVKGRFTISRDDSKNTLYLQMNSLRAEDT AVYYCVRHGNFGNSYVSWFAYWGQGTLVTVSS | 97 |
| | VL Amino acid | QAVVTQEPSLTVSPGGTVTLTCGSSTGAVTTSNYANWVQEKPGQAFR GLIGGTNKRAPGTPARFSGSLLGGKAALTLSGAQPEDEAEYYCALWY SNLWVFGGGTKLTVL | 98 |
| | VH Nucleic acid | GAGGTGCAACTGCTGGAAAGCGGCGGAGGACTCGTGCAGCCCGGC GGCAGCCTGAGACTGTCTTGTGCCGCCAGCGGATTTACATTCAGCA CCTACGCCATGAACTGGGTCAGACAGGCCCCTGGCAAGGGCCTGG AATGGGTGTCCAGAATCAGAAGCAAGTACAACAACTACGCTACATA CTATGCTGATAGCGTGAAGGGCAGATTCACCATCAGCCGGGACGAC AGCAAAAACACCCTGTACCTGCAGATGAATTCTCTGCGGGCCGAGG ACACCGCCGTGTACTACTGCGTGCGGCACGGCAACTTCGGCAATAG CTACGTGTCCTGGTTCGCCTACTGGGGCCAGGGCACCCTGGTTACA GTGTCTAGC | 99 |
| | VL Nucleic acid | CAAGCCGTGGTGACACAGGAACCCAGCCTGACCGTGTCCCCAGGC GGAACAGTGACCCTCACCTGTGGCTCTTCTACAGGCGCTGTGACCA CCAGCAACTACGCCAACTGGGTGCAGGAAAAGCCCGGCCAGGCCT TCAGAGGCCTGATCGGCGGCACAAACAAGCGGGCCCCTGGCACCC CTGCCAGATTCAGCGGCAGCCTGCTGGGCGGAAAAGCCGCTCTGA CCCTGAGCGGCGCCCAGCCTGAGGACGAGGCCGAGTACTACTGCG CCCTGTGGTATAGCAATCTGTGGGTCTTTGGCGGAGGAACCAAGCT GACAGTGCTG | 100 |
| | CD3VH-Knob | EVQLLESGGGLVQPGGSLRLSCAASGFTFSTYAMNWVRQAPGKGLE WVSRIRSKYNNYATYYADSVKGRFTISRDDSKNTLYLQMNSLRAEDT AVYYCVRHGNFGNSYVSWFAYWGQGTLVTVSSASTKGPSVFPLAPSS KSTSGGTAALGCLVKDYFPEPVTVSWNSGALTSGVHTFPAVLQSSGLY | 101 |
| | | SLSSVVTVPSSSLGTQTYICNVNHKPSNTKVDKKVEPKSCDKTHTCPP CPAPELLGGPSVFLFPPKPKDTLMISRTPEVTCVVVDVSHEDPEVKFN WYVDGVEVHNAKTKPREEQYNSTYRVVSVLTVLHQDWLNGKEYKC KVSNKALPAPIEKTISKAKGQPREPQVYTLPPCREEMTKNQVSLWCLV KGFYPSDIAVEWESNGQPENNYKTTPPVLDSDGSFFLYSKLTVDKSRW QQGNVFSCSVMHEALHNHYTQKSLSLSPGK | |
| | G05-Hole | DIQMTQSPSSLSASVGDRVTITCQASESISKFLSWYQQKPGKVPKLLIY RASTLASGVPSRFSGSGSGTDFTLTISSLQPEDVATYYCQGYDGSSTTT NYESAFGQGTKLTVLGGGGSGGGGSGGGGSEVQLVESGGGLVQPGGS LRLSCAASGFSLTDNGITWVRQAPGKGLEWIGIMTTGGSTYYAGWAK GRFTISRDNSKNTLYLQMNSLRAEDTATYFCTRGYGIHGLWGPGTLVT VSSDKTHTCPPCPAPELLGGPSVFLFPPKPKDTLMISRTPEVTCVVVDV SHEDPEVKFNWYVDGVEVHNAKTKPREEQYNSTYRVVSVLTVLHQD WLNGKEYKCKVSNKALPAPIEKTISKAKGQPREPQVCTLPPSREEMTK NQVSLSCAVKGFYPSDIAVEWESNGQPENNYKTTPPVLDSDGSFFLVS KLTVDKSRWQQGNVFSCSVMHEALHNHYTQKSLSLSPGK | 102 |
| | G07-Hole | DIQMTQSPSSLSASVGDRVTITCQASESVSKFLSWYQQKPGKVPKLLIY RASTLASGVPSRFSGSGSGTDFTLTISSLQPEDVATYYCQGYDGSSTTT NYESAFGQGTKLTVLGGGGSGGGGSGGGGSEVQLVESGGGLVQPGGS LRLSCAASGFSLTDNGITWVRQAPGKGLEWIGIMTTGGTTYYAGWAK GRFTISRDNSKNTLYLQMNSLRAEDTAVYYCARGYGIHGLWGPGTLV TVSSDKTHTCPPCPAPELLGGPSVFLFPPKPKDTLMISRTPEVTCVVVD VSHEDPEVKFNWYVDGVEVHNAKTKPREEQYNSTYRVVSVLTVLHQ DWLNGKEYKCKVSNKALPAPIEKTISKAKGQPREPQVCTLPPSREEMT KNQVSLSCAVKGFYPSDIAVEWESNGQPENNYKTTPPVLDSDGSFFLV SKLTVDKSRWQQGNVFSCSVMHEALHNHYTQKSLSLSPGK | 103 |
| | G09-Hole | DIQMTQSPSSLSASVGDRVTITCQASESISNYLSWYQQKPGKVPKLLIY RASTLASGVPSRFSGSGSGTDFTLTISSLQPEDVATYYCQGYDGSASSS NYESAFGQGTKLTVLGGGGSGGGGSGGGGSEVQLVESGGGLVQPGGS LRLSCAASGFSLSNNGITWVRQAPGKGLEWIGIITTTGSAYYAGWAKG RFTISRDNSKNTLYLQMNSLRAEDTAVYYCARGYGIHGLWGPGTLVT VSSDKTHTCPPCPAPELLGGPSVFLFPPKPKDTLMISRTPEVTCVVVDV SHEDPEVKFNWYVDGVEVHNAKTKPREEQYNSTYRVVSVLTVLHQD WLNGKEYKCKVSNKALPAPIEKTISKAKGQPREPQVCTLPPSREEMTK NQVSLSCAVKGFYPSDIAVEWESNGQPENNYKTTPPVLDSDGSFFLVS KLTVDKSRWQQGNVFSCSVMHEALHNHYTQKSLSLSPGK | 104 |
| | G00-Hole | QSALTQPASVSGSPGQSITISCTGTSSDVGGYNYVSWYQQHPGKAPKL MIYDVSKRPSGVSNRFSGSKSGNTASLTISGLQAEDEADYYCSSYTRSS TEVFGGGTKLTVLGSRGGGGSGGGGSGGGGSLEMAEVQLVQSGAEV KKPGASVKVSCKASGYTFTSYYMHWVRQAPGQGLEWMGIINPSGGS TSYAQKFQGRVTMTRDTSTSTVYMELSSLRSEDTAVYYCARGGSKKW SGEKWRRENFDYWGQGTLVTVSSDKTHTCPPCPAPELLGGPSVFLFPP KPKDTLMISRTPEVTCVVVDVSHEDPEVKFNWYVDGVEVHNAKTKP REEQYNSTYRVVSVLTVLHQDWLNGKEYKCKVSNKALPAPIEKTISK AKGQPREPQVCTLPPSREEMTKNQVSLSCAVKGFYPSDIAVEWESNGQ PENNYKTTPPVLDSDGSFFLVSKLTVDKSRWQQGNVFSCSVMHEALH NHYTQKSLSLSPGK | 105 |
| Fc | Knob | PCPAPELLGGPSVFLFPPKPKDTLMISRTPEVTCVVVDVSHEDPEVKFN WYVDGVEVHNAKTKPREEQYNSTYRVVSVLTVLHQDWLNGKEYKC KVSNKALPAPIEKTISKAKGQPREPQVYTLPPCREEMTKNQVSLWCLV KGFYPSDIAVEWESNGQPENNYKTTPPVLDSDGSFFLYSKLTVDKSRW QQGNVFSCSVMHEALHNHYTQKSLSLSPGK | 106 |
| | Hole | PCPAPELLGGPSVFLFPPKPKDTLMISRTPEVTCVVVDVSHEDPEVKFN WYVDGVEVHNAKTKPREEQYNSTYRVVSVLTVLHQDWLNGKEYKC KVSNKALPAPIEKTISKAKGQPREPQVCTLPPSREEMTKNQVSLSCAV KGFYPSDIAVEWESNGQPENNYKTTPPVLDSDGSFFLVSKLTVDKSRW QQGNVFSCSVMHEALHNHYTQKSLSLSPGK | 107 |

All the references mentioned in the present invention are cited herein as references, just as if each reference were individually cited. Additionally, it should be understood that after reading the above disclosure of the present invention, those skilled in the art may make various alterations or modifications to the invention, and such equivalent forms also fall within the scope defined by the claims of this application.

## Claims

1. An antibody or an antigen-binding fragment thereof, comprising a heavy chain variable region, wherein the heavy chain variable region comprises the following CDRs:
VH-CDR1 as shown in SEQ ID NO: 44, 78, 1, 33, 67, 22, or 87;
VH-CDR2 as shown in SEQ ID NO: 45, 59, 79, 12, 34, 68, 2, 23, 54, or 88; and
VH-CDR3 as shown in SEQ ID NO: 46, 60, 13, 35, 69, 3, 24, 55, or 89; wherein the antibody or its antigen-binding fragment specifically binds to GPRC5D, preferably human GPRC5D.

2. The antibody or antigen-binding fragment thereof of claim 1, wherein the antibody or antigen-binding fragment thereof further comprises a light chain variable region, wherein the light chain variable region comprises the following CDRs:
VL-CDR1 as shown in SEQ ID NO: 47, 61, 80, 14, 36, 70, 4, 25, 47, or 90;
VL-CDR2 as shown in SEQ ID NO: 37, 15, 71, 5, or 26; and
VL-CDR3 as shown in SEQ ID NO: 48, 81, 16, 38, 72, 6, 27, 48, or 91.

3. The antibody or antigen-binding fragment thereof of claim 1, wherein the antibody or antigen-binding fragment thereof comprises a heavy chain variable region having at least 80%, 85%, 90%, 91%, 92%, 93%, 94%, 95%, 96%, 97%, 98%, or 99% sequence identity with the amino acid sequence of SEQ ID NO: 49, 62, 82, 17, 39, 73, 7, 28, 56, or 92.

4. The antibody or antigen-binding fragment thereof of claim 2, wherein the antibody or antigen-binding fragment thereof comprises a light chain variable region having at least 80%, 85%, 90%, 91%, 92%, 93%, 94%, 95%, 96%, 97%, 98%, or 99% sequence identity with the amino acid sequence of SEQ ID NO: 51, 64, 84, 19, 41, 75, 9, 30, or 94.

5. A recombinant protein, comprising: (i) the antibody or antigen-binding fragment thereof of any one of claims 1-4; and (ii) an optional tag sequence for assisting in expression and/or purification.

6. A polynucleotide encoding a polypeptide selected from the group consisting of: (1) the antibody or antigen-binding fragment thereof of any one of claims 1-4; and (2) the recombinant protein of claim 5.

7. A vector containing the polynucleotide of claim 6.

8. An immunoconjugate, comprising: (a) the antibody or antigen-binding fragment thereof of any one of claims 1-4 or the recombinant protein of claim 5; and (b) a coupled moiety selected from the group consisting of detectable labels, drugs, toxins, cytokines, radioactive isotopes, and enzymes.

9. A chimeric antigen receptor, wherein the antigen-binding domain of the chimeric antigen receptor comprises the antibody or antigen-binding fragment thereof of any one of claims 1-4.

10. An immune cell, wherein the immune cell expresses or displays on its cell membrane the antibody or antigen-binding fragment thereof of any one of claims 1-4.

11. A bispecific antibody, comprising the antibody or antigen-binding fragment thereof of any one of claims 1-4.

12. The bispecific antibody of claim 11, wherein the bispecific antibody further comprises an antibody or an antigen-binding fragment thereof targeting CD3.

13. The use of an active ingredient selected from the group consisting of: the antibody or antigen-binding fragment thereof of any one of claims 1-4, the recombinant protein of claim 5, the immunoconjugate of claim 8, the immune cell of claim 10, the bispecific antibody of claim 11, and a combination thereof, for preparing diagnostic reagents or kits, or for preparing drugs for the treatment of diseases associated with high expression of GPRC5D.

14. A pharmaceutical composition, comprising: (z1) an active ingredient selected from the group consisting of: the antibody or antigen-binding fragment thereof of any one of claims 1-4, the recombinant protein of claim 5, the immunoconjugate of claim 8, the immune cell of claim 10, the bispecific antibody of claim 11, and a combination thereof; and (z2) a pharmaceutically acceptable carrier.

15. A method for treating diseases associated with high expression of GPRC5D, comprising administering to a subject in need thereof the antibody or antigen-binding fragment thereof of any one of claims 1-4, the recombinant protein of claim 5, the immunoconjugate of claim 8, the immune cell of claim 10, the bispecific antibody of claim 11, or the pharmaceutical composition of claim 14.
